# EUROPEAN PATENT APPLICATION

(11) **EP 4 821 484 A1**
(43) Date of publication of application: **14.10.2026**
(21) Application number: 24899791.8
(22) Date of filing: 03.12.2024
(51) Int. Cl.: A61K 9/19, C07K 19/00, C07K 16/30, C07K 16/28

(54) **MULTISPECIFIC-ANTIBODY COMPOSITION, PREPARATION METHOD THEREFOR, AND USE THEREOF**

(30) Priority: 04.12.2023 CN 202311648214
(71) Applicant: Shandong Simcere Zaiming Biopharmaceutical Co., Ltd., Yantai, Shandong 264006 (CN)
(72) Inventor: MA, Fei, Nanjing, Jiangsu 210042 (CN); LI, Xinxin, Nanjing, Jiangsu 210042 (CN); HU, Jianzhong, Nanjing, Jiangsu 210042 (CN); TANG, Renhong, Shanghai 201318 (CN)
(74) Representative: Potter Clarkson
(86) International application number: PCT/CN2024/136308
(87) International publication number: WO 2025/119153

(57) **Abstract**

The present disclosure relates to a composition, which can be a lyophilization preparation liquid or a lyophilized powder preparation. The composition comprises a multispecific antibody, a surfactant, a buffer system, and a stabilizer/a osmotic pressure regulator. Further disclosed are a preparation method and use of the composition, for example use in the treatment of a tumor, in particular multiple myeloma.

## Description

### Cross-references to related applications

The present disclosure claims the priority and related interests of the Chinese patent application No. 202311648214.5 filed on 4 December 2023, the entire content of the aforementioned Chinese patent application is incorporated herein by reference.

### Technical Field

The present disclosure relates to the field of biomedicine, and specifically to a multispecific antibody preparation and a preparation method therefor and the use thereof.

### Background

Multiple myeloma (MM) is a plasma cell malignant tumor characterized by the unrestricted proliferation of plasma cells in the bone marrow like tumor cells, accompanied by secretion of monoclonal immunoglobulins, which causes a series of clinical manifestations such as multiple osteolytic damage, hypercalcemia, anemia, kidney damage and repeated infections. Multiple myeloma accounts for 1% of all tumors and 10%-15% of hematologic malignancies. The ratio of male to female is 1.6: 1, and most patients are > 40 years old. The treatment of multiple myeloma includes chemotherapy, hematopoietic stem cell transplantation, etc.

B-cell maturation antigen (BCMA), also known as CD269 or TNFRSF17, is a member of the tumor necrosis factor receptor superfamily. BCMA is mainly expressed on the surface of mature B lymphocytes and plasma cells, and is a landmark protein for the maturation of B lymphocytes. B cell activating factor (BAFF) and proliferation inducing ligand (APRIL) are the main ligands of BCMA, which transmit cell stimulation signals by interacting with BCMA, activate TRAF-dependent NF-κB and JNK pathways and increase the proliferation and survival rate of B cells. BCMA is mainly expressed on the surface of mature B lymphocytes and plasma cells, but hardly expressed in other tissue cells. BCMA is highly expressed in tumor cells of most patients with multiple myeloma, so BCMA is an ideal therapeutic target for multiple myeloma.

GPRC5D is G protein coupled receptor C5 family subtype D, which is an orphan receptor and is a seven-transmembrane protein. GPRC5D is a new target for multiple myeloma after BCMA. It is found in tissue expression profiling studies that GPRC5D is specifically highly expressed in plasma cells of multiple myeloma, while has a low expression in normal tissues, which is limited to hair follicle regions with immune privilege. Moreover, the expression of GPRC5D does not coincide with that of BCMA. For tumor recurrence models with BCMA loss, GPRC5D CAR-T still has a therapeutic effect, overcoming tumor escape. In theory, dual-targeting both GPRC5D and BCMA could cover a larger population and bring about better clinical responses.

The inventors have previously obtained a trispecific antibody molecule targeting BCMA-GPRC5D-CD3 and have demonstrated that it has excellent anti-tumor activity. The relevant description is recorded in PCT application PCT/CN 2023/098241 and is incorporated into the present disclosure by reference in its entirety.

### Summary of the Invention

In the first aspect, the present disclosure provides a composition, comprising a multispecific antibody molecule, wherein the polyspecific antibody comprises:
(1) a first antigen-binding part that binds to the BCMA antigen of a tumor cell;
(2) The second antigen-binding part that binds to the GPRC5D antigen of tumor cells;
(3) The third antigen-binding part that binds to the CD3 antigen of immune cells.

In some embodiments, the composition is a lyophilization preparation liquid. In some embodiments, the composition comprises a multispecific antibody molecule, a buffer system and a surfactant.

In certain embodiments, the composition comprises:
(1) a multispecific antibody molecule at about 1 mg/mL to 100 mg/mL;
(2) a buffer system at about 1 mM to 50 mM; and
(3) a surfactant at about 0.01% to 0.1% (w/v);
wherein the composition has a pH not less than 5.0.

In certain embodiments, the composition has a pH of about 5.0 to 7.0.

In certain embodiments, the composition has a pH of about 5.0 to about 6.0.

In certain embodiments, the composition has a pH of about 5.5.

In some embodiments, the first antigen-binding portion of the BCMA in the multispecific antibody comprises HCDR1, HCDR2 and HCDR3 of a VHH, wherein HCDR1 contains sequences as shown in SEQ ID NO.2 and HCDR2 contains sequences as shown in SEQ ID NO.3, The HCDR3 contains a sequence as shown in SEQ ID NO.4.

In some embodiments, the second antigen-binding portion of GPRC5D in the polyspecific antibody contains heavy chain variable regions and light chain variable regions of the antibody or fragment thereof, wherein the heavy chain variable region comprises HCDR1 as shown in SEQ ID NO.6, HCDR2 as shown in SEQ ID NO.7, and HCDR3 as shown in SEQ ID NO.8; and, wherein the light chain variable region comprises LCDR1 as shown in SEQ ID NO.10, LCDR2 as shown in SEQ ID NO.11 and LCDR3 as shown in SEQ ID NO.12.

In some embodiments, the third antigen-binding portion of the multispecific antibody that binds to CD3 contains the heavy chain variable region and the light chain variable region of the antibody or fragment thereof Wherein the heavy chain variable region comprises HCDR1 as shown in SEQ ID NO.14, HCDR2 as shown in SEQ ID NO.15, and HCDR3 as shown in SEQ ID NO.16; And, wherein the light chain variable region comprises LCDR1 as shown in SEQ ID NO.18, LCDR2 as shown in SEQ ID NO.19 and LCDR3 as shown in SEQ ID NO.20.

In certain embodiments, the multispecific antibody molecule has a concentration of about 1 mg/mL to 100 mg/mL.

In certain embodiments, the multispecific antibody molecule has a concentration of about 5 mg/mL, about 10 mg/mL, about 20 mg/mL, about 30 mg/mL, about 40 mg/mL, about 50 mg/mL, about 60 mg/mL, about 70 mg/mL, about 80 mg/mL, about 90 mg/mL or about 100 mg/mL.

In certain embodiments, the buffer system has a concentration of about 1 mM to 50 mM.

In certain embodiments, the buffer system has a concentration of about 5 mM to 20 mM.

In certain embodiments, the buffer system has a concentration of about 5 mM, about 10 mM, about 15 mM, or about 20 mM.

In certain embodiments, the buffer system comprises one or more of an acetate buffer system, a citrate buffer system, a histidine salt buffer system and/or a phosphate buffer system, preferably a histidine salt buffer system.

In certain embodiments, the buffer system comprises a histidine-histidine hydrochloride buffer system, and the histidine-histidine hydrochloride buffer system has a concentration of about 5 mM to 20 mM, preferably about 5 mM.

In certain embodiments, the surfactant has a content of 0.01% to 0.1% (w/v), preferably 0.02% to 0.08%, and more preferably 0.02%, 0.04%, 0.06% or 0.08%..

In certain embodiments, the surfactant comprises polysorbate 80 and/or poloxamer 188.

In certain embodiments, the surfactant comprises polysorbate 80, and the polysorbate 80 has a content of about 0.01% to 0.1%, preferably 0.04%.

In certain embodiments, the composition further comprises a protein stabilizer, the protein stabilizer comprises sodium chloride, glycine, arginine hydrochloride, sucrose, trehalose, mannitol and/or sorbitol, preferably trehalose.

In certain embodiments, the protein stabilizer has a concentration of about 1% to 10% (w/v), preferably about 2% to 10%, more preferably about 2%, about 2.5%, about 3%, about 3.5%, about 4%, about 4.5%, about 5%, about 5.5%, about 6%, about 6.5%, about 7%, about 7.5%, about 8%, about 8.5%, about 9%, or about 9.5%.

In certain embodiments, the protein stabilizer is trehalose, and trehalose has a concentration of 2% to 10% (w/v), preferably 8% or 9.5%.

In certain embodiments, the composition further comprises an osmotic pressure regulator.

In certain embodiments, the tonicity adjusting agent comprises sodium chloride, potassium chloride, arginine hydrochloride, glycine, mannitol, sucrose, trehalose, and/or sorbitol.

In certain embodiments, the composition further comprises a pH regulator.

In certain embodiments, the pH regulator comprises acetic acid, sodium acetate, hydrochloric acid, sodium hydroxide, potassium hydroxide, citric acid, sodium citrate, sodium dihydrogen phosphate, disodium hydrogen phosphate, *etc*.

In the second aspect, the present disclosure provides a lyophilized powder preparation, which is prepared from the composition described in the first aspect after lyophilizing. In an optional embodiment, before lyophilizing, steps of sterilization and/or pyrogen removal of the composition are further comprised.

In some embodiments, the lyophilized powder preparation is reconstituted for subcutaneous injection to the patient.

In certain embodiments, the lyophilized powder preparation has a moisture content not exceed 3%.

In certain embodiments, the multispecific antibody after reconstitution of the lyophilized powder preparation has a concentration of about 5 mg/mL to 100 mg/mL, preferably about 10 mg/mL, about 20 mg/mL, about 30 mg/mL, about 40 mg/mL, about 50 mg/mL, about 60 mg/mL, about 70 mg/mL, about 80 mg/mL, about 90 mg/mL or about 100 mg/mL.

In the third aspect, the present disclosure provides a method for preparing the composition described in the first aspect (e.g., a lyophilization preparation liquid), the method comprises the following steps:
(1) thoroughly mixing the buffer component, the surfactant and the excipients such as protein stabilizers with the solvent to obtain a mixed solution;
(2) thoroughly mixing the multispecific antibody molecule and the mixed solution;
optionally, the preparation method further comprises steps of sterilization and pyrogen removal.

In the preparation method described in the present disclosure, the order of adding the buffer component, the surfactant and the adjuvant is not necessarily specified, as long as the above ingredients are fully mixed. The above ingredients can be mixed and fully dissolved in the solvent of the present disclosure to form a uniform composition.

In the fourth aspect, the present disclosure further provides a method for preparing a lyophilized powder preparation, the method comprises lyophilizing the lyophilization preparation liquid prepared according to the method described in the third aspect.

In fifth aspect, the present disclosure further provides a lyophilized powder preparation for subcutaneous injection. The lyophilized powder is prepared by lyophilizing the aforementioned lyophilization preparation liquid.

In a sixth aspect, the present disclosure also provides use of the composition according to the first aspect, or the lyophilized powder formulation according to the second or fifth aspect, in the manufacture of a medicament for treating tumors.

In a seventh aspect, the present disclosure also provides use of the composition according to the first aspect, or the lyophilized powder formulation according to the second or fifth aspect, for treating a subject suffering from a tumor.

In an eighth aspect, the present disclosure also provides a method for treating a tumor in a subject, comprising administering the composition according to the first aspect, or the lyophilized powder formulation according to the second or fifth aspect, to the subject.

In a ninth aspect, the present disclosure also provides the composition according to the first aspect, or the lyophilized powder formulation according to the second or fifth aspect, for use in treating a tumor or treating a subject suffering from a tumor.

In some embodiments, the tumor according to any one of the sixth to ninth aspects is a B-cell lymphoma. In a preferred embodiment, the B-cell lymphoma is multiple myeloma (MM). In a more preferred embodiment, the multiple myeloma is relapsed/refractory multiple myeloma (R/R MM).

In some embodiments, the present disclosure provides a lyophilized preparation liquid and a lyophilized powder formulation for a multi-specific antibody with a large molecular weight and an asymmetric structure, which exhibit excellent stability. In specific embodiments, the lyophilized powder formulation remains stable for at least 24 months under storage conditions of 2-8°C, ensuring the therapeutic efficacy of the macromolecular protein drug.

Since subcutaneous administration requires a small injection volume, preparing formulations with high concentrations of large molecular weight multi-specific antibodies presents a significant challenge. The multi-specific antibody involved in the present disclosure comprises a BCMA-GPRC5D-CD3 trispecific antibody, which has a large molecular weight. However, the present disclosure provides a formulation containing said trispecific antibody that is suitable for subcutaneous injection. The trispecific antibody shows a low propensity to aggregate at high concentrations both during the preparation of the lyophilized preparation liquid and upon reconstitution for administration, significantly improving patient compliance and/or drug accessibility.

### Brief Description of the Figures

Fig. 1 is a schematic structural diagram of the Tri-52 molecule.
Fig. 2 is a trend chart of the SEC-HPLC results of the stability test of the final formulation.
Fig. 3 is a trend chart of the CE-SDS (NR) results of the stability test of the final formulation.
Fig. 4 is a trend chart of the CE-SDS(R) results of the stability test of the final formulation.
Fig. 5 is a trend chart of the iCIEF results of the stability test of the final formulation.

### Detailed Description

Preperation of macromolecular protein drugs (such as antibodies) are typically injectables suitable for parenteral administration, and their routes of administration generally include subcutaneous, intravenous, and intramuscular injections. Since proteins are unstable in solution and highly prone to particle formation and aggregation, stability has become a major challenge in the development of macromolecular protein drugs. The multi-specific antibody (e.g., trispecific antibody) formulation of the present disclosure, whose primary active ingredient possesses a large molecular weight and a complex structure, imposes higher demands on formulation stability. Thus, developing a formulation for macromolecular protein drugs (such as antibodies) first requires addressing the issue of protein stability. To resolve the stability issues of multi-specific antibodies (e.g., trispecific antibodies), the inventors of the present disclosure have developed a lyophilized preparation liquid and a lyophilized powder formulation for multi-specific antibodies, achieving stable storage of the multi-specific antibodies (e.g., trispecific antibodies) at high concentrations and effectively preventing aggregation after reconstitution. The formulation described in the present disclosure can maintain the stability of the multi-specific antibody during long-term product storage and transportation, thereby ensuring the stability, safety, and/or efficacy of the drug product.

### Definition of Terms

Unless otherwise defined herein, scientific and technical terms related to the present disclosure shall have the meanings understood by those of ordinary skill in the art.

In addition, unless otherwise specified herein, terms in the singular form herein shall include that in the plural form, and terms in the plural form shall include that in the singular form. More specifically, as used in the description and the appended claims, the singular forms "a/an" and "this" include plural referents, unless otherwise clearly stated.

The terms "include", "comprise" and "have" are used interchangeably herein and are intended to indicate the inclusiveness of the solution, meaning that the solution can have other elements than those listed. Furthermore, it should be understood that the description of "include", "comprise" and "have" as used herein also provides the solution of "consist of". Illustratively, "a composition comprising A and B" should be understood as the following technical solution: composition consisting of A and B, and composition containing other components in addition to A and B, fall within the scope of the aforementioned "a composition".

The term "and/or" as used herein includes the meanings of "and", "or" and "all or any other combination of elements linked by the term".

The term "antibody" herein includes an intact antibody and any antigen-binding fragment (i.e., "antigen-binding moiety") or single chain thereof. The "antibody" refers to a glycoprotein comprising at least two heavy (H) chains and two light (L) chains interconnected by a disulfide bond, or an antigen binding moiety thereof. Each heavy chain consists of a heavy chain variable region (abbreviated herein as VH) and a heavy chain constant region. The heavy chain constant region consists of three domains, CH1, CH2 and CH3. Each light chain consists of a light chain variable region (abbreviated herein as VL) and a light chain constant region. The light chain constant region consists of one domain, CL. The VH and VL regions can be further subdivided into hypervariable regions known as complementarity determining regions (CDRs), which are interspersed among more conserved regions known as framework regions (FRs). Each VH and VL consists of three CDRs and four FRs, which are arranged in the following order from amino terminus to carboxyl terminus: FR1, CDR1, FR2, CDR2, FR3, CDR3, FR4. The variable regions of the heavy and light chains contain a binding domain that can interact with an antigen. The constant region of an antibody can mediate the binding of an immunoglobulin to a host tissue or factor, and the host tissue or factor includes various cells of the immune system (e.g., effector cells) and a first component of the classical complement system (C1q). Since the amino acid composition and arrangement sequence of the heavy chain constant region of an immunoglobulin are different, the antigenicity of the immunoglobulin is also different. Accordingly, "immunoglobulin" herein can be divided into five classes, or isotypes of immunoglobulins, namely IgM, IgD, IgG, IgA and IgE, and the corresponding heavy chains thereof are a µ chain, a δ chain, a γ chain, an α chain and an ε chain, respectively. The same class of Ig can also be divided into different subclasses according to the differences in the amino acid composition of the hinge region thereof and the number and position of heavy chain disulfide bonds. For example, IgG can be divided into IgG1, IgG2, IgG3 and IgG4, and IgA can be divided into IgA1 and IgA2. The light chains are divided into a κ chain or a λ chain by the difference in the constant region. Each Ig class of the five Ig classes can have either a κ chain or a λ chain.

"Antibody" herein also includes an antibody that does not comprise light chains, for example, heavy-chain antibodies (HCAbs) produced from camelids, such as Camelus dromedarius, Camelus bactrianus, Lama glama, Lama guanicoe and Vicugna pacos, and Ig new antigen receptors (IgNARs) found in Chondrichthyes such as sharks.

The term "antibody" herein can be derived from any animals, including but not limited to humans and non-human animals. The non-human animals can be selected from primates, mammals, rodents and vertebrates, such as camelids, Lama glama, Lama guanicoe, Vicugna pacos, goats, rabbits, mice, rats or Chondrichthyes (such as sharks).

The term "heavy chain antibody" herein refers to an antibody that lacks light chains of a conventional antibody. The term specifically includes, but is not limited to, a homodimeric antibody comprising a VH antigen binding domain and CH2 and CH3 constant domains in the absence of a CH1 domain.

The term "nanobody" herein refers to a natural heavy chain antibody without light chains in camel, *etc*., and cloning the variable region thereof can obtain a single domain antibody, also known as VHH (Variable domain of heavy chain of heavy chain antibody), which only consists of a heavy chain variable region, and is the smallest functional antigen-binding fragment.

The terms "VHH domain", "nanobody" and "single domain antibody" (sdAb) herein have the same meaning and are used interchangeably, and refer to a single domain antibody consisting of only one heavy chain variable region constructed by cloning the variable region of a heavy chain antibody, which is the smallest antigen-binding fragment with full functionality. Generally, a single domain antibody consisting of only one heavy chain variable region is constructed by obtaining a heavy chain antibody that naturally lacks light chains and heavy chain constant region 1 (CH1), and then cloning the heavy chain variable region of the antibody.

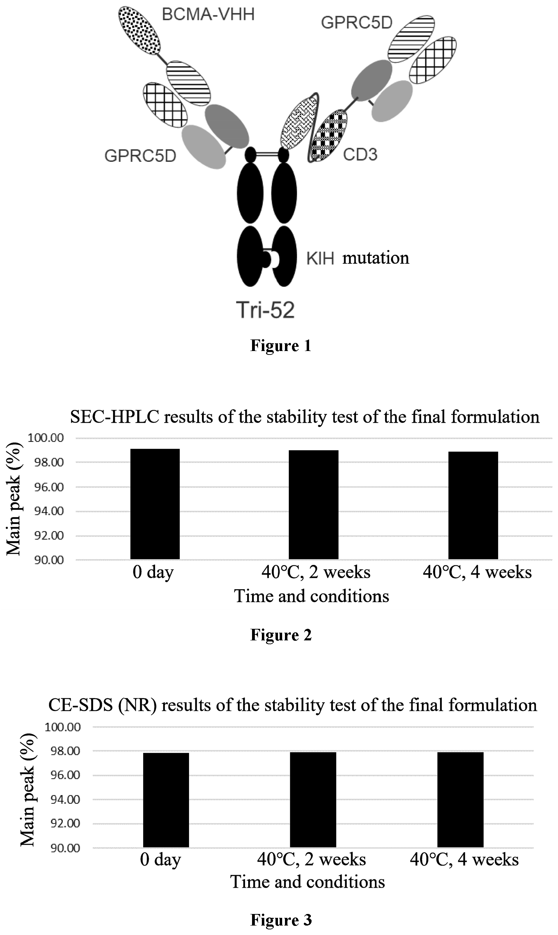

The term "multispecific" herein refers to the ability of an antibody or an antigen-binding fragment to specifically bind to, for example, different antigens or at least two different epitopes on the same antigen. Therefore, the terms such as "bispecific", "trispecific" and "tetraspecific" refer to the number of different epitopes to which an antibody can bind. For example, a conventional monospecific IgG-type antibody has two identical antigen binding sites (paratopes) and thus can only bind to the same epitope (rather than bind to different epitopes). In contrast, a multispecific antibody has at least two different types of paratopes/binding sites and thus can bind to at least two different epitopes. As described herein, "complementarity determining region" refers to an antigen binding site of an antibody. Furthermore, single "specific" may refer to one, two, three or more identical complementarity determining regions in a single antibody (the actual number of complementarity determining regions/binding sites in a single antibody molecule is referred to as "valent"). For example, a single natural IgG antibody is monospecific and bivalent because it has two identical paratopes. Accordingly, a multispecific antibody comprises at least two (different) complementarity determining regions/binding sites. Therefore, the term "multispecific antibody" refers to an antibody that has more than one paratope and has the ability to bind to two or more different epitopes. The term "multispecific antibody" particularly includes the bispecific antibody as defined above, and generally also includes a protein, e.g., an antibody or a scaffold specifically binding to three or more different epitopes, i.e., an antibody having three or more paratopes/binding sites. Exemplarily, the multispecific antibody comprised in the composition of the present disclosure is a trispecific antibody targeting BCMA-GPRC5D-CD3, and has a structure as shown in Fig. 1.

The term "valence" herein refers to the presence of a defined number of binding sites in an antibody/an antigen binding molecule. Therefore, the terms "monovalent", "bivalent", "tetravalent" and "hexavalent" indicate the presence of one binding site, two binding sites, four binding sites and six binding sites in an antibody/antigen binding molecule, respectively. Exemplarily, the trispecific antibody comprised in the composition of the present disclosure is tetravalent and comprises one binding site for binding to BCMA, two binding sites for binding to GPRCC5D, and one binding site for binding to CD3.

The "antigen binding fragment" and "antibody fragment" are used interchangeably herein, which do not possess the full structure of an intact antibody, and only comprise a part of an intact antibody or a variant of the part, wherein the part of the intact antibody or the variant of the part has the ability to bind to an antigen. Exemplarily, "antigen-binding fragment" or "antibody fragment" herein includes, but is not limited to, Fab, F(ab')₂, Fab', Fab'-SH, Fd, Fv, scFv, diabody and a single domain antibody.

The term "variable region" herein refers to a region in the heavy or light chain of an antibody that is involved in enabling the antibody to bind to an antigen. "Heavy chain variable region", "VH" and "HCVR" are used interchangeably, and "light chain variable region", "VL" and "LCVR" are used interchangeably. The variable domains of the heavy and light chains of a natural antibody generally have similar structures, and each domain comprises four conserved framework regions (FRs) and three hypervariable regions (HVRs).

The terms "complementarity determining region" and "CDR" are used interchangeably herein and generally refer to hypervariable regions (HVRs) found in both light and heavy chain variable domains. The more conservative portion of a variable domain is called the framework region (FR). As understood in the art, the amino acid positions representing the hypervariable region of an antibody may vary according to the context and various definitions known in the art. Some positions within variable domains can be considered heterozygous hypervariable positions because these positions can be considered to be within the hypervariable regions under one set of criteria (such as IMGT or KABAT) but outside the hypervariable regions under a different set of criteria (such as KABAT or IMGT). One or more of these positions may also be found in extended hypervariable regions. The disclosure includes an antibody comprising modifications in these hybrid hypervariable positions. The heavy chain variable region CDR can be abbreviated as HCDR, and the light chain variable region CDR can be abbreviated as LCDR. The variable domains of a natural heavy chain and light chain each comprise four framework regions predominantly in a sheet configuration, which are linked by three CDRs (CDR1, CDR2 and CDR3) that form a loop linking the sheet structure, and in some cases form part of the sheet structure. The CDRs in each chain are closely held together in the order of FR1-CDR1-FR2-CDR2-FR3-CDR3-FR4 by the FR region, and together with the CDRs from other antibody chains, contribute to the formation of an antigen binding site of an antibody.

The "CDRs" herein can be marked and defined by well-known methods in the art, including but not limited to Kabat numbering system, Chothia numbering system or IMGT numbering system. The tool websites used include, but are not limited to, AbRSA website (http://cao.labshare.cn/AbRSA/cdrs.php), abYsis website (www.abysis.org/abysis/sequence_input /key_annotation/key_annotation.cgi) and IMGT website (http://www.imgt.org/3Dstructure-DB/cgi/ DomainGapAlign.cgi#results). The CDRs herein include overlaps and subsets of amino acid residues defined in different ways.

The term "heavy chain constant region" herein refers to the carboxyl terminus portion of an antibody heavy chain, which is not directly involved in the binding of an antibody to an antigen, but exhibits an effector function, such as an interaction with an Fc receptor, and has a more conservative amino acid sequence relative to the variable domain of an antibody. The "heavy chain constant region" can be selected from a CH1 domain, a hinge region, a CH2 domain, a CH3 domain, or a variant or fragment thereof. The "heavy chain constant region" includes a "full-length heavy chain constant region" and a "heavy chain constant region fragment", the former has a structure substantially similar to that of a natural antibody constant region, while the latter only includes "a portion of the full-length heavy chain constant region". Exemplarily, a typical "full-length antibody heavy chain constant region" consists of CH1 domain-hinge region-CH2 domain-CH3 domain, which also includes a CH4 domain when an IgE antibody is referred to, and does not include the CH1 domain when a heavy chain antibody is referred to. Exemplarily, a typical "heavy chain constant region fragment" can be selected from Fc or a CH3 domain.

The term "light chain constant region" herein refers to the carboxyl terminus portion of an antibody light chain, which is not directly involved in the binding of an antibody to an antigen, and the light chain constant region can be selected from a constant κ domain or a constant λ domain.

The term "Fc region" herein is used to define the C-terminal region of an antibody heavy chain that contains at least a portion of the constant region. The term includes a native sequence Fc region and a variant Fc region. Exemplarily, human IgG heavy chain Fc region can extend from Cys226 or Pro230 to the carboxyl terminus of the heavy chain. However, an antibody generated from a host cell may undergo post-translational cleavage whereby one or more, particularly one or two amino acids are cleaved off from the C-terminus of the heavy chain. Therefore, by expression of a specific nucleic acid molecule encoding a full-length heavy chain, the antibody generated from a host cell can include the full-length heavy chain, or a cleavage variant of the full-length heavy chain. This may be the case when the last two C-terminal amino acids of the heavy chain are glycine (G446) and lysine (K447, numbering according to the Kabat EU index). Therefore, the C-terminal lysine (Lys447), or the C-terminal glycine (Gly446) and lysine (Lys447) of the Fc region may or may not be present. Typically, an IgG Fc region comprises IgG CH2 and IgG CH3 domains, and optionally, on this basis, the IgG Fc region can also comprise a complete or partial hinge region, but does not comprise a CH1 domain. The "CH2 domain" of a human IgG Fc region generally extends from an amino acid residue at about position 231 to an amino acid residue at about position 340. Unless otherwise specified herein, the numbering of amino acid residues in the Fc region or constant region follows the EU numbering system, also known as the EU index.

The term "Fc variant" herein refers to changes in the structure or function of Fc caused by one or more amino acid substitution, insertion or deletion mutations at appropriate positions on the Fc. The "interaction between Fc variants" refers to the space-filling effect, electrostatic steering, hydrogen bonding effect, hydrophobic effect, etc. formed between Fc variants that have been subjected to a mutation design. The interaction between Fc variants contributes to the formation of a stable heterodimeric protein. A preferred mutation design is a mutation design in a "Knob-into-Hole" form.

The mutation design technique of Fc variants has been widely used in the field to prepare bispecific antibodies or heterodimeric Fc fusion protein forms. Representative ones include the "Knob-into-Hole" form proposed by Cater *et al*.; the Fc-containing heterodimer form formed by using Electrostatic Steering by the technician in Amgen (US 20100286374 A1); the heterodimer form (SEEDbodies) formed by IgG/IgA chain exchange proposed by Jonathan H. Davis *et al*.; the bispecific molecules formed by Genmab's DuoBody platform technology; the heterodimeric protein forms formed by combining structural calculation and Fc amino acid mutations and combining different modes of action by Xencor's technical staff; the heterodimeric protein form obtained by the charge network-based Fc modification method of Alphamab Oncology (CN 201110459100.7); and other genetic engineering methods for forming heterodimeric functional proteins based on Fc amino acid changes or functional modification means. The Knob/Hole structure on the Fc variant fragments of the present disclosure means that the two Fc fragments are mutated respectively, and can be combined in a "Knob-into-Hole" form after the mutations. Preferably, the Fc regions are subjected to site mutation modification using the "Knob-into-hole" model of Cater et al., so that the obtained first Fc variant and second Fc variant can be combined together in a "Knob-into-hole" form to form a heterodimer. The selection of particular immunoglobulin Fc regions from particular immunoglobulin classes and subclasses is within the range of those skilled in the art. The Fc regions of human antibodies IgG1, IgG2, IgG3 and IgG4 are preferred, and the Fc region of human antibody IgG1 is more preferred. Either of the first Fc variant or the second Fc variant is randomly selected for knob mutation and the other for hole mutation.

The term "effector function" herein refers to the activity of an antibody molecule, which is mediated by the binding of the domain of the antibody rather than the antigen binding domain, usually mediated by the binding of an effector molecule. Effector functions include complement-mediated effector functions, which are mediated by, for example, the binding of the C1 component of the complement to the antibody. The activation of a complement is important in the conditioning and lysis of cell pathogens. The activation of a complement also stimulates inflammatory response and can participate in autoimmune hypersensitivity response. Effector functions also include Fc receptor (FcR)-mediated effector functions, which can be triggered by the binding of the constant domain of the antibody to the Fc receptor (FcR). The binding of antibodies to Fc receptors on the cell surface triggers many important and diverse biological responses, including phagocytosis and destruction of antibody-coated particles, clearance of immune complexes, lysis of antibody-coated target cells by killer cells (called antibody-dependent cell-mediated cytotoxicity, or ADCC), release of inflammatory mediators, placental transfer, and control of immunoglobulin production. The effector functions of an antibody can be changed by, for example, enhancing or decreasing the affinity of the antibody to an effector molecule such as an Fc receptor or a complement component. Generally, the binding affinity will be changed by modifying the binding site of an effector molecule, and in this case, it is appropriate to locate the site of interest and modify at least part of the site in an appropriate manner. It is also assumed that changes in binding sites on an antibody against an effector molecule do not need to significantly change the overall binding affinity, but may change the geometry of the interaction, resulting in the invalid effector mechanism, as in unproductive binding. It is further assumed that effector functions can also be changed by modifying sites that are not directly involved in the binding of effector molecules but otherwise participate in the effector functions.

The term "identity" herein can be calculated by the following method. To determine the percent "identity" of two amino acid sequences or two nucleic acid sequences, the sequences are aligned for optimal comparison purposes (for example, gaps can be introduced in either or both of the first and second amino acid sequences or nucleic acid sequences for optimal alignment or non-homologous sequences can be discarded for comparison purposes). The amino acid residues or nucleotides at corresponding amino acid positions or nucleotide positions are then compared. When the position in the first sequence is occupied by the same amino acid residue or nucleotide as that at the corresponding position in the second sequence, the molecules are identical at that position. The percent identity between two sequences will vary with the identical positions shared by the sequences, considering the number of gaps that need to be introduced to optimally align the two sequences and the length of each gap.

The sequence comparison and the calculation of percent identity between two sequences can be achieved using a mathematical algorithm. For example, the percent identity between two amino acid sequences is determined using the Needleman and Wunsch algorithm in the GAP program that has been integrated into the GCG software package (available at www.gcg.com) and using the Blossum 62 matrix or the PAM250 matrix, with a gap weight of 16, 14, 12, 10, 8, 6 or 4 and a length weight of 1, 2, 3, 4, 5 or 6. For another example, the percent identity between two nucleotide sequences is determined using the GAP program in the GCG software package (available at www.gcg.com) and using the NWSgapdna.CMP matrix, with a gap weight of 40, 50, 60, 70 or 80 and a length weight of 1, 2, 3, 4, 5 or 6. A particularly preferred parameter set (and one parameter set that should be used unless otherwise stated) is the Blossum62 scoring matrix with a gap penalty of 12, a gap extension penalty of 4, and a gap frameshift penalty of 5. The percent identity between two amino acid sequences or nucleotide sequences can also be determined using the PAM120 weighted remainder table, with a gap length penalty of 12 and a gap penalty of 4, and using the E. Meyers and W. Miller algorithm that has been incorporated into the ALIGN program (version 2.0).

The term "linker" herein refers to linking polypeptide sequences for linking a protein domain, and generally has a certain degree of flexibility. The use of linker does not result in loss of the original function of the protein domain. Exemplary linker includes (G₄S)₃.

The terms "composition" and "preparation" herein refer to a product comprising a multispecific antibody described in the present disclosure in a predetermined amount or ratio, as well as any product produced directly or indirectly by combining a predetermined amount of a multispecific antibody described in the present disclosure. The meaning of the composition or preparation described in the present disclosure may comprise a pharmaceutical preparation, that is, a product comprising the multispecific antibody, the adjuvant, the buffer component and the surfactant, and any product directly or indirectly produced by the combination, compounding or aggregation of any two or more of the ingredients. The preparation may be in the form of a solution or a lyophilized powder.

The term "buffer component" herein refers to an agent that has the function of providing a buffering effect (for example, resistance to changes in pH). For example, the buffer component can adjust the change of pH due to the addition and/or release of acidic or alkaline substances. For example, the buffer component may comprise a weak acid and its conjugate base; the buffer component may also comprise a weak base and its conjugate acid.

"Buffer" or "buffer solution" as used herein generally refers to an aqueous solution containing a mixture of an acid (usually a weak acid, such as acetic acid, citric acid, the hydrochloride form of histidine) and its conjugate base (such as acetate or a citrate salt, such as sodium acetate, sodium citrate, or histidine), or a mixture of a base (usually a weak base, such as histidine) and its conjugate acid (such as a protonated histidine salt). Due to the "buffering effect" imparted by the "buffer", the pH of the "buffer solution" changes only slightly when a small amount of strong acid or alkali is added.

The term "pH regulator" herein comprises a substance that adjusts pH. The term "pH regulator" also refers to a variety of pH regulators. The term "pH regulator" refers to one pH regulator or two or more pH regulators. Therefore, the term "pH regulator" also comprises a mixture comprising or consisting of different pH regulators. Examples of the pH regulator comprise acetic acid, sodium acetate, hydrochloric acid, sodium hydroxide, potassium hydroxide, citric acid, sodium citrate, sodium dihydrogen phosphate, and disodium hydrogen phosphate.

The term "surfactant" herein refers to an agent that can protect a protein (such as the multispecific antibody of the present disclosure) from the stress induced by air/solution interface and the stress induced by solution/surface to reduce the aggregation of the protein or minimize the formation of particles in the composition. The surfactant may comprise both hydrophobic groups and hydrophilic groups. The surfactant may comprise a mixture or combination of one or more surfactants. The surfactant may comprise a nonionic surfactant or an ionic surfactant.

The term "nonionic surfactant" herein refers to a surfactant containing a hydrophilic group that does not dissociate in an aqueous solution. For example, the nonionic surfactant may not dissociate into an ionic state in an aqueous solution, but may exist in the solution in a molecular or micellar state. For example, the nonionic surfactant may comprise a polyoxyethylene type nonionic surfactant. (such as fatty alcohol polyoxyethylene ether, alkylphenol polyoxyethylene ether (OP), octylphenol polyoxyethylene ether-10, dodecylphenol polyoxyethylene ether or fatty acid polyoxyethylene ether), polyol type nonionic surfactant (such as a span-type surfactant, a Tween-type surfactant, polysorbates) and/or polyethers (such as fully embedded polyethers). The nonionic surfactant can have high stability, acid, alkali and ions have little influence on the nonionic surfactant, and/or the nonionic surfactant has strong hard water resistance.

The term "osmotic pressure regulator" herein refers to an agent used to adjust the osmotic pressure of a liquid preparation. For example, the osmotic pressure regulator can make the osmotic pressure of a liquid preparation containing it isotonic with body fluids (for example, human plasma) to avoid damaging tissue. Examples of the osmotic pressure regulator herein comprise sodium chloride, potassium chloride, arginine hydrochloride, glycine, mannitol, sucrose, trehalose and/or sorbitol.

In the present disclosure, the term "solvent" generally refers to a liquid that has the ability to dissolve other substances. The solvent may comprise an organic solvent (such as aromatic hydrocarbons, aliphatic hydrocarbons, alicyclic hydrocarbons, halogenated hydrocarbons, alcohols, ethers, esters or ketones) and an inorganic solvent (such as water). For example, the water may comprise pure water, that is, H₂O without impurities. For example, the water may comprise water for injection, that is, water that complies with the provisions of water for injection in the Chinese Pharmacopoeia. The water for injection may comprise distilled water or water obtained by distillation of deionized water.

In the present disclosure, the terms "stability" and "stable" refer to the resistance of an antibody in a formulation to aggregation, degradation or fragmentation under given manufacturing, preparation, transportation and storage conditions. A "stable" formulation retains biological activity under given manufacturing, preparation, transportation and storage conditions. Compared to a reference formulation, for example, by appearance, pH value, concentration, purity (molecular exclusion chromatography (SEC-HPLC), non-reduced sodium dodecyl sulfate capillary electrophoresis (CE-SDS(NR), reduced sodium dodecyl sulfate capillary electrophoresis (CE-SDS(R)), charge isomerism (full-column capillary isoelectric focusing electrophoresis (iCIEF)), particle size detected by dynamic light scattering (DLS) and binding activity (detected by ELISA). The detection methods are usually carried out in accordance with the latest version of the Chinese Pharmacopoeia and corresponding guidelines.

In the present disclosure, the term "about" refers to a range of 0.5%-10% above or below a specified value, for example, a range of about 0.5%, about 1%, about 1.5%, about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5% or about 10% above or below a specified value. For example, for the surfactant, the concentration is about 0.01% to 0.1%, and the percentage value is allowed to be within the error range allowed in this field, such as ± 0.01%; For the protein stabilizer, the concentration is about 1% to 10%, and the percentage value is allowed to be within the error range allowed in this field, such as ± 0.5%.

In the present disclosure, when referencing the concentration or content of surfactants, excipients such as stabilizers, and the like, it refers to mass concentration, which may also be expressed as w/v.

The term "administer" and similar terms herein are generally not limited to physical administration; suitable methods include *in vitro*, *ex vivo* or *in vivo* methods. For example, any method of administration known to those skilled in the art for contacting cells, organs or tissues with the composition may be employed. For example, the compound may be introduced into the body of a subject in need of treatment by any route of introduction or delivery. In some embodiments, the composition of the present disclosure is administered primarily subcutaneously.

The term "treatment" herein refers to surgical or therapeutic treatment, the purpose of which is to prevent and slow down (reduce) an undesired physiological change or pathology in a subject being treated, such as cancers and tumors. Beneficial or desired clinical outcomes include, but are not limited to, the alleviation of symptoms, the weakening of disease severity, the stabilization of disease state (i.e., no deterioration), the delay or slowing down of disease progression, the amelioration or mitigation of disease state, and remission (whether partial or complete), whether detectable or undetectable. Objects in need of treatment include those who already have a disorder or disease, those who are susceptible to a disorder or disease or those who intend to prevent a disorder or disease. When terms such as slowing down, alleviation, weakening, mitigation, and remission are referred to, their meanings also include elimination, disappearance, non-occurrence and other circumstances.

The term "subject" refers to an organism receiving treatment for a particular disease or condition as described herein. Exemplarily, a "subject" includes a mammal, such as a human, a primate (e.g., monkey) or a non-primate mammal, receiving treatment for a disease or a condition.

The term "effective amount" herein refers to an amount of a therapeutic agent effective to prevent or relieve a disease or a disorder or the progression of the disease when administered alone or in combination with another therapeutic agent to a cell, tissue or subject. The "effective amount" also refers to an amount of a compound sufficient to relieve symptoms, such as treating, curing, preventing or relieving related medical disorders, or increasing the speed of treating, curing, preventing or relieving these disorders. When the active ingredient is administered to an individual alone, a therapeutically effective dose refers to the ingredient alone. When a certain combination is applied, a therapeutically effective dose refers to the combined dosage of the active ingredients that produce a therapeutic effect, whether administered in combination, sequentially or simultaneously.

The term "cancer" herein refers to or describes the physiological condition in mammals that is typically characterized by unregulated cell growth. Both benign and malignant cancers are included in this definition. The term "tumor" or "neoplasm" herein refers to all neoplastic cell growth and proliferation, whether malignant or benign, and to all pre-cancerous and cancerous cells and tissues. The terms "cancer" and "tumor" are not mutually exclusive when referred to herein.

The present disclosure is further described below in conjunction with specific embodiments and examples, from which the advantages and features of the present disclosure will become more apparent.

### Compositions

In one aspect, the present disclosure provides a composition, which is a lyophilization preparation liquid and comprises a multispecific antibody, a buffer component and a surfactant.

In some embodiments, the composition comprises a multispecific antibody at a concentration of about 1 mg/mL to 100 mg/mL.

In some embodiments, the multispecific antibody has a concentration of about 5 mg/mL to 100 mg/mL.

In some embodiments, the multispecific antibody has a concentration of about 5 mg/mL, about 10 mg/mL, about 20 mg/mL, about 30 mg/mL, about 40 mg/mL, about 50 mg/mL, about 60 mg/mL, about 70 mg/mL, about 80 mg/mL, about 90 mg/mL or about 100 mg/mL, or any concentration between any two of the above-mentioned values.

In some embodiments, the multispecific antibody comprises:
(1) a first antigen-binding moiety that binds to tumor cell BCMA antigen;
(2) a second antigen-binding moiety that binds to tumor cell GPRC5D antigen;
(3) a third antigen-binding moiety that binds to immune cell CD3 antigen.

In some embodiments, the multispecific antibody comprises a first light chain, a second light chain, a first heavy chain and a second heavy chain, wherein:
(1) the first light chain and the second light chain are the same and have the structure as shown below: VLₐ-CL; and
(2) the first heavy chain and the second heavy chain are different and have the structure as shown below: the first heavy chain comprises VHH_{b}-L1-VHₐ-CH1-Fc1 and the second heavy chain comprises VHₐ-CH1-L2-VH_{CD3}-L3-VL_{CD3}-Fc2; optionally, the carboxyl terminus of one heavy chain further has a Flag tag and/or the carboxyl terminus of the other heavy chain further has a His tag;
wherein VHa and VLa are a heavy chain variable region and a light chain variable region that specifically bind to GPRC5D, respectively, VHHb is a nanobody that specifically binds to BCMA,VHCD3 and VLCD3 are a heavy chain variable region and a light chain variable region that specifically bind to CD3, respectively, and L1, L2 and L3 are the same or different linkers, respectively.

In some embodiments, the first heavy chain has a structure of VHHb-L1-VHa-CH1-Fc1. In some embodiments, the second heavy chain has a structure of VHa-CH1-L2-VHCD3-L3-VLCD3-Fc2.

In some embodiments, the Fc1 and the Fc2 of the multispecific antibody are different, wherein the Fc1 is a knob-Fc and the Fc2 is a hole-Fc; or, the Fc1 is a hole-Fc, and the Fc2 is a knob-Fc; preferably, wherein the knob-Fc comprises a T366W mutation, and/or the hole-Fc comprises a T366S, L368A and/or Y407V mutation; preferably, the Fc1 and the Fc2 are the Fc region of IgG4; and more preferably, the Fc1 is a knob-Fc and the Fc2 is a hole-Fc.

In some embodiments, the Fc1 and the Fc2 of the multispecific antibody are different and have an amino acid mutation that changes an effector function; preferably, the mutation that changes the effector function comprises a S228P, F234A and/or L235A mutation of IgG4 Fc.

In some embodiments, in the multispecific antibody:
(1) the first antigen-binding moiety targeting BCMA comprises HCDR1, HCDR2 and HCDR3 of VHH, wherein the HCDR1 comprises a sequence as shown in SEQ ID NO.2, the HCDR2 comprises a sequence as shown in SEQ ID NO.3, and the HCDR3 comprises a sequence as shown in SEQ ID NO.4;
(2) the second antigen-binding moiety targeting GPRC5D comprises a heavy chain variable region and a light chain variable region of the antibody or a fragment thereof, the heavy chain variable region comprises HCDR1 with a sequence as shown in SEQ ID NO. 6, HCDR2 with a sequence as shown in SEQ ID NO.7, and HCDR3 with a sequence as shown in SEQ ID NO.8; and the light chain variable region comprises LCDR1 with a sequence as shown in SEQ ID NO.10, LCDR2 with a sequence as shown in SEQ ID NO.11, and LCDR3 with a sequence as shown in SEQ ID NO.12; and
(3) the third antigen-binding moiety targeting CD3 comprises a heavy chain variable region and a light chain variable region of the antibody or a fragment thereof, the heavy chain variable region comprises HCDR1 with a sequence as shown in SEQ ID NO. 14, HCDR2 with a sequence as shown in SEQ ID NO.15, and HCDR3 with a sequence as shown in SEQ ID NO.16; and the light chain variable region comprises LCDR1 with a sequence as shown in SEQ ID NO.18, LCDR2 with a sequence as shown in SEQ ID NO.19, and LCDR3 with a sequence as shown in SEQ ID NO.20.

In some embodiments, the multispecific antibody comprises:
(1) the first antigen-binding moiety targeting BCMA comprising a VHH with a sequence as shown in SEQ ID NO. 1;
(2) the second antigen-binding moiety targeting GPRC5D comprising a heavy chain variable region with a sequence as shown in SEQ ID NO.5 and a light chain variable region with a sequence as shown in SEQ ID NO.9; and
(3) the third antigen-binding moiety targeting CD3 comprising a heavy chain variable region with a sequence as shown in SEQ ID NO.13 and a light chain variable region with a sequence as shown in SEQ ID NO.17.

In some embodiments, the multispecific antibody comprises the Fc domain of human IgG4.

In some embodiments, the human IgG4 Fc domain comprises a Fc1 and a Fc2 which are different.

In some embodiments, the Fc1 and the Fc2 which are different comprise an amino acid mutation that changes an effector function, wherein the mutation that changes the effector function comprise a S228P, F234A and/or L235A mutation of IgG4 Fc.

In some embodiments, the Fc1 and the Fc2 which are different comprise a heterodimerization-promoting amino acid mutation, and the heterodimerization-promoting amino acid mutation comprises a knob-into-hole (KIH) mutation; and more preferably, the Knob-into-hole mutation comprises: (a) a T366W mutation in either Fc region; and (b) a T366S, L368A and/or Y407V mutation in the other Fc region.

In some embodiments, the multispecific antibody is trispecific antibody, wherein the trispecific antibody comprises a first heavy chain with a sequence as shown in SEQ ID NO.21, a second heavy chain with a sequence as shown in SEQ ID NO.22, a first light chain with a sequence as shown in SEQ ID NO.23 and a second light chain with a sequence as shown in SEQ ID NO.23.

In some embodiments, the either Fc region further comprises a His-tag, and the other Fc region further comprises a Flag-tag.

In some embodiments, the composition has a pH of about 5.0 to 7.0.

In some embodiments, the composition has a pH of about 5.0 to 6.5.

In some embodiments, the composition has a pH of about 5.0 to 6.0.

In some embodiments, the composition has a pH of about 5.5 to 6.0.

In some embodiments, the composition has a pH of about 5.5.

In some embodiments, the buffer component comprises a citrate buffer solution, a histidine buffer solution and/or a phosphate buffer solution. In a preferred embodiment, the buffering component is a histidine salt buffer solution, for example, a histidine-histidine hydrochloride buffer solution.

In some embodiments, the buffer component has a content of about 1 to 50 mM, about 1 to 45 mM, about 1 to 40 mM, about 1 to 35 mM, about 1 to 20 mM, about 1 to 15 mM, about 1 to 10 mM, about 1 to 5 mM, about 5 to 50 mM, about 5 to 45 mM, about 5 to 40 mM, about 5 to 35 mM, about 5 to 30 mM, about 5 to 25 mM or about 5 to 20 mM. In a preferred embodiment, the content of the histidine-hydrochloride buffer solution is about 5-20 mM, and more preferably 5 mM.

In some embodiments, the buffer component comprises histidine-histidine hydrochloride buffer solution, and the histidine-hydrochloride buffer solution can have a content of about 1 to 50 mM, about 1 to 45 mM, about 1 to 40 mM, about 1 to 35 mM, about 1 to 20 mM, about 1 to 15 mM, about 1 to 10 mM, about 1 to 5 mM, about 5 to 50 mM, about 5 to 45 mM, about 5 to 40 mM, about 5 to 35 mM, about 5 to 30 mM, about 5 to 25 mM or about 5 to 20 mM.

In some embodiments, the buffer component has a content of about 1 mM, about 2 mM, about 3 mM, about 4 mM, about 5 mM, about 10 mM, about 15 mM, about 20 mM, about 25 mM, about 30 mM, about 35 mM, about 40 mM, about 45 mM or about 50 mM, or any concentration between any two of the above-mentioned values.

In some embodiments, the buffer component comprises histidine-histidine hydrochloride buffer solution, and the histidine-hydrochloride buffer solution can have a content of about 1 mM, about 2 mM, about 3 mM, about 4 mM, about 5 mM, about 10 mM, about 15 mM, about 20 mM, about 25 mM, about 30 mM, about 35 mM, about 40 mM, about 45 mM or about 50 mM, or any concentration between any two of the above-mentioned values.

In some embodiments, the surfactant comprises polysorbate 80 and/or poloxamer 188. In a preferred embodiment, the surfactant is polysorbate 80.

In some embodiments, the surfactant comprises polysorbate 80, and the polysorbate 80 can have a mass concentration content of about 0.01% to 1%, about 0.01 to 0.5%, about 0.01 to 0.1%, about 0.01 to 0.08%, about 0.01 to 0.06%, about 0.01 to 0.04%, about 0.01 to 0.02%, about 0.02 to 1%, about 0.04 to 1%, about 0.02 to 0.5%, about 0.02 to 0.1% or about 0.02 to 0.08%. In a preferred embodiment, the mass concentration of the polysorbate 80 is approximately 0.02-0.08%, and more preferably 0.04%.

In some embodiments, the surfactant comprises polysorbate 80, and the polysorbate 80 can have a mass concentration content of about 0.2%, 0.4%, 0.6% or 0.8%, or any concentration between any two of the above-mentioned values.

In some embodiments, the surfactant comprises poloxamer 188, and the poloxamer 188 can have a mass concentration content of about 0.01% to 1%, about 0.01 to 0.5%, about 0.01 to 0.1%, about 0.01 to 0.08%, about 0.01 to 0.06%, about 0.01 to 0.04%, about 0.01 to 0.02%, about 0.02 to 1%, about 0.04 to 1%, about 0.02 to 0.5%, about 0.02 to 0.1% or about 0.02 to 0.08%.

In some embodiments, the surfactant comprises poloxamer 188, and the poloxamer 188 can have a mass concentration content of about 0.2%, 0.4%, 0.6%, or 0.8%, or any concentration between any two of the above-mentioned values.

In some embodiments, the composition further comprises an excipient. In a preferred embodiment, the excipient is a stabilizer, such as a protein stabilizer.

In some embodiments, the composition further comprises a stabilizer. For example, the composition further comprises a stabilizer, and the stabilizer comprises glycine, sucrose, trehalose and/or mannitol. In a preferred embodiment, the stabilizer is trehalose or sucrose.

In a more preferred embodiment, the stabilizer is trehalose. In some embodiments, within a reasonable concentration range, trehalose can improve the stability of the formulation; thus, trehalose is the most preferred excipient.

In some embodiments, the stabilizer has a content of about 1% to 10%, about 1% to 9.5%, about 1% to 9.0%, about 1% to 8.5%, about 1% to 8.0%, about 1% to 7.5%, about 1% to 7.0%, about 1% to 6.5%, about 1% to 6.0%, about 1% to 5.5%, about 1% to 5.0%, about 1% to 4.5%, about 1% to 4%, about 1% to 3.5%, about 1% to 3%, about 1% to 2.5%, about 1% to 2%, about 2% to 10%, about 2% to 9.5%, about 2% to 9.0%, about 2% to 8.5%, about 2% to 8.0%, about 2% to 7.5%, about 2% to 7.0%, about 2% to 6.5%, about 2% to 6.0%, about 2% to 5.5%, about 2% to 5.0% or about 2% to 4.5%.

In some embodiments, the stabilizer has a content of about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5% or about 10%, or any concentration between any two of the above-mentioned values.

In some embodiments, the stabilizer comprises trehalose, and the trehalose has a content of about 1% to 10%, about 1% to 9.5%, about 1% to 9.0%, about 1% to 8.5%, about 1% to 8.0%, about 1% to 7.5%, about 1% to 7.0%, about 2% to 10%, about 2% to 9.5%, about 2% to 9.0%, about 2% to 8.5%, about 2% to 8.0%, about 2% to 7.5%, about 2% to 7.0%, about 4.5% to 10%, about 4.5% to 9.5%, about 4.5% to 9.0%, about 4.5% to 8.5%, about 4.5% to 8.0%, about 4.5% to 7.5%, or about 4.5% to 7.0%. In a preferred embodiment, the mass concentration of the trehalose is about 8.0%-9.5%, and more preferably 9.5%.

In some embodiments, the stabilizer comprises trehalose, and the trehalose has a content of about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5% or about 10%, or any concentration between any two of the above-mentioned values.

In some embodiments, the stabilizer comprises glycine, and the glycine has a content of about 1% to 5.0%, about 1% to 4.5%, about 1% to 4.0%, about 1% to 3.5%, about 1% to 3.0%, about 1% to 2.5%, about 1% to 2.0%, about 1.5% to 5.0%, about 1.5% to 4.5%, about 1.5% to 4.0%, about 1.5% to 3.5%, about 1.5% to 3.0%, about 1.5% to 2.5% or about 1.5% to 2.0%.

In some embodiments, the stabilizer comprises glycine, and the glycine can have a content of about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5% or about 10%, or any concentration between any two of the above-mentioned values.

In some embodiments, the stabilizer comprises sucrose, and the sucrose has a content of about 1% to 10%, about 1% to 9.5%, about 1% to 9.0%, about 1% to 8.5%, about 1% to 8.0%, about 1% to 7.5%, about 1% to 7.0%, about 2% to 10%, about 2% to 9.5%, about 2% to 9.0%, about 2% to 8.5%, about 2% to 8.0%, about 2% to 7.5%, about 2% to 7.0%, about 4.5% to 10%, about 4.5% to 9.5%, about 4.5% to 9.0%, about 4.5% to 8.5%, about 4.5% to 8.0%, about 4.5% to 7.5% or about 4.5% to 7.0%.

In some embodiments, the stabilizer comprises sucrose, and the sucrose has a content of about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5% or about 10%, or any concentration between any two of the above-mentioned values.

In some embodiments, the stabilizer comprises mannitol, and the mannitol has a content of about 1% to 10%, about 1% to 9.5%, about 1% to 9.0%, about 1% to 8.5%, about 1% to 8.0%, about 1% to 7.5%, about 1% to 7.0%, about 1% to 6.5%, about 1% to 6.0%, about 1% to 5.5%, about 1% to 5.0%, about 1% to 4.5%, about 2% to 10%, about 2% to 9.5%, about 2% to 9.0%, about 2% to 8.5%, about 2% to 8.0%, about 2% to 7.5%, about 2% to 7.0%, about 2% to 6.5%, about 2% to 6.0%, about 2% to 5.5%, about 2% to 5.0%, or about 2% to 4.5%.

In some embodiments, the stabilizer comprises mannitol, and the mannitol can have a content of about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5% or about 10%, or any concentration between any two of the above-mentioned values.

In some embodiments, the composition comprises:
(1) a multi-specific antibody;
(2) about 5-20 mM histidine-histidine hydrochloride buffer system;
(3) about 0.02%-0.08% mass concentration of polysorbate 80; and
(4) about 8.0%-9.5% mass concentration of trehalose;
wherein the pH of the composition is about 5.0-7.5.

In some embodiments, the composition comprises:
(1) a multi-specific antibody;
(2) about 5 mM histidine-histidine hydrochloride buffer system;
(3) about 0.04% mass concentration of polysorbate 80; and
(4) about 9.5% mass concentration of trehalose;
wherein the pH of the composition is about 5.5.

In some embodiments, the composition comprises:
(1) a multispecific antibody at about 1 mg/mL to 100 mg/mL;
(2) a buffer component at about 5 to 20 mM;
(3) a surfactant at about 0.02% to 0.08% mass concentration; and
(4) a stabilizer at about 1% to 10% mass concentration;
wherein the composition may have a pH of about 5.0 to 7.5.

In some embodiments, the composition comprises:
(1) a multispecific antibody at about 1 mg/mL to 100 mg/mL;
(2) a histidine-histidine hydrochloride buffer system at about 5 mM to 20 mM;
(3) polysorbate 80 at about 0.02% to 0.08% mass concentration; and
(4) trehalose at about 1% to 10% mass concentration;
wherein the composition may have a pH of about 5.0 to 7.5.

In some embodiments, the composition comprises:
(1) a multispecific antibody at about 10 mg/mL;
(2) a histidine-histidine hydrochloride buffer system at about 5 mM;
(3) polysorbate 80 at about 0.04% (w/v); and
(4) trehalose at about 9.5% (w/v);
wherein the composition has a pH of about 5.5.

In some embodiments, the composition further comprises a solvent. For example, the solvent comprises a sterile pure water and/or water for injection.

In another aspect, the present disclosure provides a lyophilized powder preparation, which is obtained by lyophilizing the aforementioned lyophilization preparation liquid;
optionally, before lyophilizing, steps of sterilization and pyrogen removal of the lyophilization preparation liquid are further comprised.

In some embodiments, the lyophilized powder preparation of the present disclosure is reconstituted and used for subcutaneous administration to a patient.

### Preparation method

In one aspect, the present disclosure provides a method for preparing the aforementioned lyophilization preparation liquid, the method comprises the following steps:
(1) thoroughly mixing the buffer component, the surfactant and the adjuvant with the solvent to obtain a mixed solution;
(2) thoroughly mixing the multispecific antibody molecule and the mixed solution;
optionally, the preparation method further comprises steps of sterilization and pyrogen removal.

In the preparation method described in the present disclosure, the order of adding the buffer component, the surfactant and the adjuvant is not necessarily specified, as long as the above ingredients are fully mixed. The above ingredients can be mixed and fully dissolved in the solvent of the present disclosure to form a uniform composition.

In another aspect, the present disclosure further provides a method for preparing a lyophilized powder preparation, the method comprises lyophilizing the lyophilization preparation liquid prepared above.

### Therapeutic use

The present disclosure provides use of the composition or the lyophilized powder formulation described in the present disclosure in the manufacture of a medicament for treating a tumor.

The present disclosure also provides use of the composition or the lyophilized powder formulation described in the present disclosure for treating a subject suffering from a tumor.

In some embodiments, the tumor is a B-cell lymphoma. In a preferred embodiment, the B-cell lymphoma is multiple myeloma (MM). In a more preferred embodiment, the multiple myeloma is relapsed/refractory multiple myeloma (R/R MM).

### Examples

The examples of the present disclosure are only exemplary and do not limit the scope of the present disclosure in any way. It should be understood by those skilled in the art that the details and forms of the technical solutions of the present disclosure could be modified or substituted without departing from the spirit and scope of the present disclosure, but these modifications and substitutions all fall within the scope of protection of the present disclosure. Where specific conditions are not specified in the examples, the procedures are carried out according to conventional conditions or conditions recommended by the manufacturer. Where the manufacturers of the reagents or instruments used are not specified, they are all conventional products that can be obtained through commercial purchase.

In the present disclosure, the BCMA-GPRC5D-CD3 multispecific antibody is as disclosed in PCT/CN 2023/098241, which is incorporated herein by reference in its entirety.

### Example 1. Preparation of trispecific antibody

### 1.1 Discovery of trispecific antibody molecule

In previous research, a trispecific antibody Tri-52 (PCT/CN 2023/098241) targeting BCMA, GPRC5D and CD3 has been developed, and has a structure as shown in Fig. 1. The sequences of the CDRs of the parts targeting BCMA, GPRC5D and CD3 and the variable region are as shown in Table 1, and the CDRs are defined according to Kabat numbering.

**Table 1 Amino acid sequences of antibody molecules**

| **Designation of sequence** | **Amino acid sequence** | **SEQ ID NO.** |
|---|---|---|
| BCMA-VHH | EVQLVESGGGLVQPGGSLRLSCAASESISSIHIMAWYRQAPGKQRELV AGIRNDGSTVYVDSVKGRFTISRDNAKNSLYLQMNSLRAEDTAVYY CNADQGFGSYSEWERRSRWGQGTTVTVSS | 1 |
| BCMA-HCDR1 | IHIMA | 2 |
| BCMA-HCDR2 | GIRNDGSTVYVDSVKG | 3 |
| BCMA-HCDR3 | DQGFGSYSEWERRSR | 4 |
| GPRC5D-VH | EVQLVQSGAEVKKPGSSVKVSCKASGYTFTSYGISWVRQAPGQGLE WMGEIYPRSGNTYYNEKFKGRVTLTADKSTSTAYMELSSLRSEDTAV YYCARGRAYGRNYPMDYWGQGTTVTVSS | 5 |
| GPRC5D-HCDR1 | SYGIS | 6 |
| GPRC5D-HCDR2 | EIYPRSGNTYYNEKFKG | 7 |
| GPRC5D-HCDR3 | GRAYGRNYPMDY | 8 |
| GPRC5D-VL | EIVLTQSPATLSLSPGERATLSCRASQSIGTSIHWYQQKPGQSPRLLIK YASESISGIPARFSGSGSGTDFTLTISSLEPEDFAVYYCQQSNSWPYTF GGGTKVEIK | 9 |
| GPRC5D-LCDR1 | RASQSIGTSIH | 10 |
| GPRC5D-LCDR2 | YASESIS | 11 |
| GPRC5D-LCDR3 | QQSNSWPYT | 12 |
| CD3-VH | EVQLVESGGGVVQPGRSLRLSCAASGFTFTDYYMSWVRQAPGKGL EWVAMSRNKAKGHTIEYSSSVKGRFTISRDNSKNTLYLQMNSLRAE DTAVYYCARDTYESYDGYFDVWGQGTTVTVSS | 13 |
| CD3-HCDR1 | DYYMS | 14 |
| CD3-HCDR2 | MSRNKAKGHTIEYSSSVKG | 15 |
| CD3-HCDR3 | DTYESYDGYFDV | 16 |
| CD3-VL | DIVMTQSPDSLAVSLGERATINCKSSQSLFNSRSRKNYLAWYQQKPG QTPKLLIYWASIRESGVPDRFSGSGSGTDFTLTISSLQAEDVAVYYCK QSYYLYTFGQGTKLEIK | 17 |
| CD3-LCDR1 | KSSQSLFNSRSRKNYLA | 18 |
| CD3-LCDR2 | WASIRES | 19 |
| CD3-LCDR3 | KQSYYLYT | 20 |
| HC-Hole | EVQLVQSGAEVKKPGSSVKVSCKASGYTFTSYGISWVRQAPGQGLE WMGEIYPRSGNTYYNEKFKGRVTLTADKSTSTAYMELSSLRSEDTAV YYCARGRAYGRNYPMDYWGQGTTVTVSSASTKGPSVFPLAPCSRST SESTAALGCLVKDYFPEPVTVSWNSGALTSGVHTFPAVLQSSGLYSLS SVVTVPSSSLGTKTYTCNVDHKPSNTKVDKRVGGGGSGGGGSGGG GSEVQLVESGGGVVQPGRSLRLSCAASGFTFTDYYMSWVRQAPGK GLEWVAMSRNKAKGHTIEYSSSVKGRFTISRDNSKNTLYLQMNSLR AEDTAVYYCARDTYESYDGYFDVWGQGTTVTVSSGGGGSGGGGSG GGGSDIVMTQSPDSLAVSLGERATINCKSSQSLFNSRSRKNYLAWYQ QKPGQTPKLLIYWASIRESGVPDRFSGSGSGTDFTLTISSLQAEDVAV YYCKQSYYLYTFGQGTKLEIKESKYGPPCPPCPAPEAAGGPSVFLFPP KPKDTLMISRTPEVTCVVVDVSQEDPEVQFNWYVDGVEVHNAKTK PREEQFNSTYRVVSVLTVLHQDWLNGKEYKCKVSNKGLPSSIEKTIS KAKGQPREPQVYTLPPSQEEMTKNQVSLSCAVKGFYPSDIAVEWES NGQPENNYKTTPPVLDSDGSFFLVSRLTVDKSRWQEGNVFSCSVMH EALHNHYTQKSLSLSLGK | 21 |
| HC-Knob | EVQLVESGGGLVQPGGSLRLSCAASESISSIHIMAWYRQAPGKQREL VAGIRNDGSTVYVDSVKGRFTISRDNAKNSLYLQMNSLRAEDTAVYY CNADQGFGSYSEWERRSRWGQGTTVTVSSGGGGSGGGGSGGGGS EVQLVQSGAEVKKPGSSVKVSCKASGYTFTSYGISWVRQAPGQGLE WMGEIYPRSGNTYYNEKFKGRVTLTADKSTSTAYMELSSLRSEDTAV YYCARGRAYGRNYPMDYWGQGTTVTVSSASTKGPSVFPLAPCSRST SESTAALGCLVKDYFPEPVTVSWNSGALTSGVHTFPAVLQSSGLYSLS SVVTVPSSSLGTKTYTCNVDHKPSNTKVDKRVESKYGPPCPPCPAPE AAGGPSVFLFPPKPKDTLMISRTPEVTCVVVDVSQEDPEVQFNWYV DGVEVHNAKTKPREEQFNSTYRVVSVLTVLHQDWLNGKEYKCKVSN KGLPSSIEKTISKAKGQPREPQVYTLPPSQEEMTKNQVSLWCLVKG FYPSDIAVEWESNGQPENNYKTTPPVLDSDGSFFLYSRLTVDKSRWQ EGNVFSCSVMHEALHNHYTQKSLSLSLGK | 22 |
| LC | EIVLTQSPATLSLSPGERATLSCRASQSIGTSIHWYQQKPGQSPRLLIK YASESISGIPARFSGSGSGTDFTLTISSLEPEDFAVYYCQQSNSWPYTF GGGTKVEIKRTVAAPSVFIFPPSDEQLKSGTASVVCLLNNFYPREAKV QWKVDNALQSGNSQESVTEQDSKDSTYSLSSTLTLSKADYEKHKVYA CEVTHQGLSSPVTKSFNRGEC | 23 |
| linker | GGGGSGGGGSGGGGS | 24 |

### 1.2 Expression and purification of trispecific antibody

The plasmid loaded with the antibody encoding gene was transfected into Expi293F cells (Thermofisher, catalog No. A14527). Six days after transfection, the cell supernatant was collected. After collecting the culture supernatant, the protein was purified by Protein A affinity and molecular sieve using AKTA Pure, and sterilized and filtered with a 0.22 µm filter. The obtained antibody was quantitatively and qualitatively analyzed through SDS-PAGE, SEC-HPLC and CE-SDS to obtain a Tri-52 trispecific antibody molecule with higher purity.

### 1.3 Binding experiments of trispecific antibody

FACS was used to identify the binding activity of the trispecific antibody molecule with tumor cells endogenously expressing BCMA and GPRC5D. The positive control antibodies were a bispecific antibody REGN5459 (WO 2020018820A1, targeting antigen BCMA and CD3 receptor expressed on T cell surface) from Regeneron and a bispecific antibody JNJ7564 (WO 2019220368A1, targeting antigen GPRC5D and CD3 receptor expressed on T cell surface) from Johnson & Johnson, and the negative control antibody was antiFITC × CD3-Hab01 (prepared internally). The results are as shown in Table 2. The results show that the trispecific antibody molecules can strongly bind to an endogenous tumor cell NCI-H929 expressing antigens BCMA and GPRC5D.

**Table 2: FACS detection of binding activity of trispecific antibody with H929 cells**

| **Designation of antibody** | **H929** | |
|---|---|---|
| | **Maximum mean fluorescence intensity** | **EC50 (nM)** |
| Tri-52 | 29813 | 3.65 |
| REGN5459 | 19901 | 61.73 |
| JNJ7564 | 1052 | 30.24 |
| antiFITC × CD3-Hab01 | 95 | Negative |

### Example 2. Buffer system screening

### 2.1 Buffer system screening scheme

The screening experiment covered 3 buffer salts (citrate, histidine salt and phosphate), 9 preparation buffer systems (pH values from 5.0 to 7.5), Tri-52 protein was used in each formulation, and the protein had a concentration of 10 mg/mL; The protein stock solution was subjected to ultrafiltration, concentration and liquid exchange, and the stock solution was subjected to exchange into the corresponding 9 buffer systems. Through the accelerated test at 40°C, the stability of this protein in 9 buffer systems was comprehensively investigated and compared with the appearance, pH value, concentration, purity (molecular exclusion chromatography (SEC-HPLC), non-reduced sodium dodecyl sulfate capillary electrophoresis (CE-SDS(NR), reduced sodium dodecyl sulfate capillary electrophoresis (CE-SDS(R)), charge isomerism (full-column capillary isoelectric focusing electrophoresis (iCIEF)), particle size detected by dynamic light scattering (DLS) and activity (binding activity detected by ELISA) as indicators, so as to screen the optimal buffer system. The specific formulation composition and investigation scheme are shown in Table 3.

The detection methods related to various indicators of the preparations of the present disclosure are all conventional detection methods well known to those skilled in the art, for example, with reference to the standards of Part III of the "Chinese Pharmacopoeia (2020 Edition)" and related guiding principles.

**Table 3 Experimental scheme for screening of preparation buffer system**

| No. | Buffer system, pH | | T0 | 40°C | | |
|---|---|---|---|---|---|---|
| | | | | 1W | 2W | 4W |
| F1 | 20 mM citric acid-sodium citrate | 5 | X | X | X | X |
| F2 | | 5.5 | X | X | X | X |
| F3 | | 6 | X | X | X | X |
| F4 | 20 mM histidine-histidine hydrochloride | 5.5 | X | X | X | X |
| F5 | | 6 | X | X | X | X |
| F6 | | 6.5 | X | X | X | X |
| F7 | 20 mM disodium hydrogen phosphate-sodium dihydrogen phosphate | 6.5 | X | X | X | X |
| F8 | | 7 | X | X | X | X |
| F9 | | 7.5 | X | X | X | X |

| | | | | | | |
|---|---|---|---|---|---|---|
| Remark: X = Appearance, pH, concentration, iCIEF, SEC-HPLC, CE-SDS(NR), CE-SDS(R), DLS, and activity. T0 = starting point of investigation; 1W = 1 week, and so on. | | | | | | |

### 2.2 Results of buffer system screening

The main results of buffer system screening are summarized in Table 4, Table 5 and Table 6.

**Table 4 Result 1 of 40°C accelerated test for preparation buffer system screening**

| No. | Investigation conditions | Appearance | Protein concentration (mg/mL) | Particle size (nm) | pH |
|---|---|---|---|---|---|
| F1 | T0 | Colorless, slight opalescence, no visible particles | 10.37 | 14.38 | 5.066 |
| | 40°C, 1 week | Colorless, severe opalescence, a large amount of precipitation | 0.76 | 491.78 | 5.047 |
| | 40°C, 2 weeks | Colorless liquid, severe opalescence, a large amount of precipitation | N/A | N/A | NA |
| | 40°C, 4 weeks | Colorless liquid, severe opalescence, a large amount of precipitation | N/A | N/A | NA |
| F2 | T0 | Colorless, slight opalescence, no visible particles | 10.44 | 14.69 | 5.514 |
| | 40°C, 1 week | Colorless, severe opalescence, a large amount of precipitation | 0.50 | 894.36 | 5.537 |
| | 40°C, 2 weeks | Colorless liquid, severe opalescence, precipitation | N/A | N/A | NA |
| | 40°C, 4 weeks | Colorless liquid, severe opalescence, a large amount of precipitation | N/A | N/A | NA |
| F3 | T0 | Colorless, slight opalescence, no visible particles | 10.41 | 14.72 | 5.967 |
| | 40°C, 1 week | Colorless, severe opalescence, a large amount of precipitation | 1.40 | 239.03 | 5.990 |
| | 40°C, 2 weeks | Colorless liquid, severe opalescence, precipitation | N/A | N/A | NA |
| | 40°C, 4 weeks | Colorless liquid, severe opalescence, a large amount of precipitation | N/A | N/A | NA |
| F4 | T0 | Colorless, slight opalescence, no visible particles | 10.41 | 12.60 | 5.573 |
| | 40°C, 1 week | Colorless, slight opalescence, no visible particles | 10.49 | 21.95 | 5.603 |
| | 40°C, 2 weeks | Colorless liquid, opalescence, no visible particles | 10.55 | 24.40 | 5.551 |
| | 40°C, 4 weeks | Colorless liquid, opalescence, no visible particles | 10.58 | 26.33 | 5.583 |
| F5 | T0 | Colorless, slight opalescence, no visible particles | 10.40 | 13.06 | 6.120 |
| | 40°C, 1 week | Colorless, slight opalescence, no visible particles | 10.46 | 24.21 | 6.160 |
| | 40°C, 2 weeks | Colorless liquid, opalescence, no visible particles | 10.47 | 27.53 | 6.065 |
| | 40°C, 4 weeks | Colorless liquid, opalescence, no visible particles | 10.53 | 31.26 | 6.183 |
| F6 | T0 | Colorless, slight opalescence, no visible particles | 10.20 | 14.21 | 6.570 |
| | 40°C, 1 week | Colorless, slight opalescence, no visible particles | 9.89 | 29.35 | 6.622 |
| | 40°C, 2 weeks | Colorless liquid, opalescence, no visible particles | 10.10 | 30.77 | 6.550 |
| | 40°C, 4 weeks | Colorless liquid, opalescence, no visible particles | 10.34 | 43.13 | 6.641 |
| F7 | T0 | Colorless, slight opalescence, no visible particles | 10.55 | 15.91 | 6.523 |
| | 40°C, 1 week | Colorless, severe opalescence, no visible particles | 7.75 | 27.97 | 6.551 |
| | 40°C, 2 weeks | Colorless liquid, severe opalescence, sticky precipitate at the bottom | 6.34 | 46.71 | 6.515 |
| | 40°C, 4 weeks | Colorless liquid, severe opalescence, a large amount of precipitation | N/A | N/A | NA |
| F8 | T0 | Colorless, slight opalescence, no visible particles | 11.95 | 17.49 | 6.986 |
| | 40°C, 1 week | Colorless, severe opalescence, no visible particles | 9.95 | 19.41 | 7.011 |
| | 40°C, 2 weeks | Colorless liquid, severe opalescence, sticky precipitate at the bottom | 8.74 | 17.93 | 7.001 |
| | 40°C, 4 weeks | Colorless liquid, severe opalescence, a large amount of precipitation | N/A | N/A | NA |
| F9 | T0 | Colorless, slight opalescence, no visible particles | 10.98 | 16.88 | 7.433 |
| | 40°C, 1 week | Colorless, severe opalescence, no visible particles | 9.76 | 18.64 | 7.416 |
| | 40°C, 2 weeks | Colorless liquid, severe opalescence, sticky precipitate at the bottom | 9.06 | 17.53 | 7.458 |
| | 40°C, 4 weeks | Colorless liquid, severe opalescence, a large amount of precipitation | N/A | N/A | NA |

| | | | | | |
|---|---|---|---|---|---|
| Remark: T0 = starting point of investigation; N/A = not applicable | | | | | |

**Table 5 Result 2 of 40°C accelerated test for preparation buffer system screening**

| No. | Investigation conditions | SEC-HPLC(%) | | | CE-SDS (NR) (%) | | |
|---|---|---|---|---|---|---|---|
| | | Low molecular weight peak | Main peak | High molecular weight peak | Low molecular weight peak | Main peak | High molecular weight peak |
| F1 | T0 | 3.6 | 80.4 | 16.0 | 2.89 | 96.51 | 0.60 |
| | 40°C, 1 week | N/A | N/A | N/A | N/A | N/A | N/A |
| | 40°C, 2 weeks | N/A | N/A | N/A | N/A | N/A | N/A |
| | 40°C, 4 weeks | N/A | N/A | N/A | N/A | N/A | N/A |
| F2 | T0 | 3.8 | 77.3 | 19.0 | 3.40 | 95.95 | 0.66 |
| | 40°C, 1 week | N/A | N/A | N/A | N/A | N/A | N/A |
| | 40°C, 2 weeks | N/A | N/A | N/A | N/A | N/A | N/A |
| | 40°C, 4 weeks | N/A | N/A | N/A | N/A | N/A | N/A |
| F3 | T0 | 3.8 | 75.6 | 20.6 | 3.45 | 96.20 | 0.34 |
| | 40°C, 1 week | N/A | N/A | N/A | N/A | N/A | N/A |
| | 40°C, 2 weeks | N/A | N/A | N/A | N/A | N/A | N/A |
| | 40°C, 4 weeks | N/A | N/A | N/A | N/A | N/A | N/A |
| F4 | T0 | 4.1 | 85.5 | 10.4 | 3.49 | 96.14 | 0.37 |
| | 40°C, 1 week | 2.4 | 89.6 | 8.0 | 4.52 | 95.07 | 0.41 |
| | 40°C, 2 weeks | 5.1 | 86.2 | 8.7 | 6.87 | 92.76 | 0.36 |
| | 40°C, 4 weeks | 9.3 | 84.9 | 5.8 | 4.64 | 95.00 | 0.37 |
| F5 | T0 | 4.1 | 84.7 | 11.1 | 3.58 | 96.03 | 0.39 |
| | 40°C, 1 week | 0.3 | 87.6 | 12.1 | 4.09 | 95.55 | 0.36 |
| | 40°C, 2 weeks | 2.0 | 84.6 | 13.5 | 4.87 | 94.78 | 0.36 |
| | 40°C, 4 weeks | 3.4 | 85.8 | 10.7 | 4.38 | 94.82 | 0.80 |
| F6 | T0 | 4.2 | 83.9 | 12.0 | 3.57 | 96.07 | 0.36 |
| | 40°C, 1 week | 1.7 | 83.9 | 14.4 | 3.92 | 95.67 | 0.41 |
| | 40°C, 2 weeks | 1.1 | 82.9 | 16.0 | 4.91 | 94.71 | 0.38 |
| | 40°C, 4 weeks | 2.5 | 83.8 | 13.7 | 3.76 | 96.24 | ND |
| F7 | T0 | 3.9 | 76.4 | 19.7 | 3.06 | 96.59 | 0.35 |
| | 40°C, 1 week | 1.9 | 74.4 | 23.7 | 2.33 | 97.37 | 0.31 |
| | 40°C, 2 weeks | N/A | N/A | N/A | N/A | N/A | N/A |
| | 40°C, 4 weeks | N/A | N/A | N/A | N/A | N/A | N/A |
| F8 | T0 | 4.4 | 75.4 | 20.3 | 2.90 | 96.75 | 0.35 |
| | 40°C, 1 week | 3.0 | 73.0 | 23.9 | 2.66 | 97.01 | 0.34 |
| | 40°C, 2 weeks | N/A | N/A | N/A | N/A | N/A | N/A |
| | 40°C, 4 weeks | N/A | N/A | N/A | N/A | N/A | N/A |
| F9 | T0 | 4.3 | 75.5 | 20.2 | 3.16 | 96.50 | 0.34 |
| | 40°C, 1 week | 4.2 | 73.0 | 22.8 | 4.92 | 94.82 | 0.26 |
| | 40°C, 2 weeks | N/A | N/A | N/A | N/A | N/A | N/A |
| | 40°C, 4 weeks | N/A | N/A | N/A | N/A | N/A | N/A |

| | | | | | | | |
|---|---|---|---|---|---|---|---|
| Remark: T0 = starting point of investigation; N/A = not applicable; ND = not detected | | | | | | | |

**Table 6 Result 3 of 40°C accelerated test for preparation buffer system screening**

| No. | Investigation conditions | CE-SDS (R) (%) | | Activit y% | iCIEF (%) | | | |
|---|---|---|---|---|---|---|---|---|
| | | Main peak | Others | | Isoele ctric point | Acidic peak | Main peak | Basic peak |
| F1 | T0 | 98.97 | 1.03 | 99 | 8.357 | 34.9 | 59.2 | 5.8 |
| | 40°C, 1 week | N/A | N/A | N/A | N/A | N/A | N/A | N/A |
| | 40°C, 2 weeks | N/A | N/A | N/A | N/A | N/A | N/A | N/A |
| | 40°C, 4 weeks | N/A | N/A | N/A | N/A | N/A | N/A | N/A |
| F2 | T0 | 98.70 | 1.30 | 105 | 8.382 | 31.4 | 63.2 | 5.4 |
| | 40°C, 1 week | N/A | N/A | N/A | N/A | N/A | N/A | N/A |
| | 40°C, 2 weeks | N/A | N/A | N/A | N/A | N/A | N/A | N/A |
| | 40°C, 4 weeks | N/A | N/A | N/A | N/A | N/A | N/A | N/A |
| F3 | T0 | 98.93 | 1.07 | 106 | 8.365 | 31.4 | 62.9 | 5.6 |
| | 40°C, 1 week | N/A | N/A | N/A | N/A | N/A | N/A | N/A |
| | 40°C, 2 weeks | N/A | N/A | N/A | N/A | N/A | N/A | N/A |
| | 40°C, 4 weeks | N/A | N/A | N/A | N/A | N/A | N/A | N/A |
| F4 | T0 | 98.62 | 1.38 | 103 | 8.403 | 32.9 | 61.2 | 5.8 |
| | 40°C, 1 week | 97.55 | 2.45 | 55 | 8.389 | 44.5 | 31.1 | 24.4 |
| | 40°C, 2 weeks | 96.23 | 3.77 | 50 | 8.339 | 40.1 | 27.1 | 32.8 |
| | 40°C, 4 weeks | 98.00 | 2.00 | 29 | 8.346 | 52.1 | 16.9 | 30.9 |
| F5 | T0 | 98.75 | 1.25 | 109 | 8.392 | 31.4 | 63.0 | 5.5 |
| | 40°C, 1 week | 97.90 | 2.10 | 72 | 8.336 | 44.2 | 42.1 | 13.8 |
| | 40°C, 2 weeks | 96.56 | 3.44 | 52 | 8.348 | 56.2 | 28.9 | 14.9 |
| | 40°C, 4 weeks | 99.23 | 0.77 | 38 | 8.339 | 67.1 | 19.3 | 13.8 |
| F6 | T0 | 98.75 | 1.25 | 104 | 8.402 | 34.8 | 59.6 | 5.6 |
| | 40°C, 1 week | 98.08 | 1.92 | 83 | 8.355 | 46.4 | 48.7 | 4.9 |
| | 40°C, 2 weeks | 97.32 | 2.68 | 64 | 8.331 | 55.4 | 34.4 | 10.3 |
| | 40°C, 4 weeks | 99.31 | 0.69 | 48 | 8.336 | 69.5 | 21.4 | 9.1 |
| F7 | T0 | 98.21 | 1.79 | 102 | 8.365 | 32.1 | 62.3 | 5.5 |
| | 40°C, 1 week | 98.04 | 1.96 | 99 | 8.319 | 46 | 48.8 | 5.3 |
| | 40°C, 2 weeks | N/A | N/A | N/A | N/A | N/A | N/A | N/A |
| | 40°C, 4 weeks | N/A | N/A | N/A | N/A | N/A | N/A | N/A |
| F8 | T0 | 98.64 | 1.36 | 102 | 8.369 | 33.6 | 60.5 | 6.0 |
| | 40°C, 1 week | 98.62 | 1.38 | 91 | 8.309 | 48 | 46 | 6.1 |
| | 40°C, 2 weeks | N/A | N/A | N/A | N/A | N/A | N/A | N/A |
| | 40°C, 4 weeks | N/A | N/A | N/A | N/A | N/A | N/A | N/A |
| F9 | T0 | 98.94 | 1.06 | 99 | 8.372 | 35.3 | 59.2 | 5.5 |
| | 40°C, 1 week | 98.57 | 1.43 | 93 | 8.312 | 52.3 | 41.5 | 6.1 |
| | 40°C, 2 weeks | N/A | N/A | N/A | N/A | N/A | N/A | N/A |
| | 40°C, 4 weeks | N/A | N/A | N/A | N/A | N/A | N/A | N/A |

| | | | | | | | | |
|---|---|---|---|---|---|---|---|---|
| Remark: T0 = starting point of investigation; N/A = not applicable | | | | | | | | |

### 2.3 Conclusion of buffer system screening

This buffer system screening experiment covered 3 types of buffer salts (citrate, histidine salt and phosphate) and 9 preparation buffer systems (pH values from 5.0 to 7.5). A 40°C high temperature accelerated test was conducted to comprehensively investigate and compare the stability of this protein in 9 buffer systems. The results are as follows:
**Appearance:** After investigation from T0 to 4 weeks at 40°C, F1 to F3 (citrate buffer system) and F7 to F9 (phosphate buffer system) all appeared a large amount of precipitation, and F4 to F6 (histidine buffer system) changed from a slightly opalescent liquid to an opalescent liquid, but did not have visible particles; Formulations with a large amount of precipitation would not be investigated later.
**pH and concentration:** The formulations of F4 to F6 were all within the target value without significant changes.
**SEC-HPLC:** After investigation at 40°C for 4 weeks, the quality of low molecular weight peaks was F6 > F5 > F4, and the quality of high molecular weight peaks: F4 > F5 > F6.
**CE-SDS (NR):** After investigation at 40°C for 4 weeks, the purity of the main peak of CE(NR) was F6 > F4 and F5; the quality of low molecular weight peaks: F6 > F4 and F5.
**CE-SDS (R):** After investigation at 40°C for 4 weeks, the content of main peaks (light chain and heavy chain) was ranked as follows: F6 and F5 > F4.
**iCIEF:** After investigation at 40°C for 4 weeks, the main peaks all showed a significant decrease.
**DLS:** After investigation at 40°C for 4 weeks, the particle size was ranked as follows: F4 > F5 > F6.
**Binding activity:** After investigation at 40°C for 4 weeks, F4, F5, and F6 all showed a significant decrease.

In summary, after 4 weeks of screening of the buffer systems, the formulations F4 to F6 (histidine buffer system) were significantly better than other formulations. For each indicator, the three formulations with different pH values of the histidine buffer system had their own advantages and disadvantages. Therefore, it was determined that the histidine buffer system was the optimal buffer system. There was no optimal pH. All the experimental pH values could be used to prepare antibody preparations. The intermediate pH formulation (F5) was tentatively determined for the next step of adjuvant screening research. The histidine buffer system would be further studied in the follow-up study.

### Example 3. Adjuvant screening

### 3.1 Adjuvant screening scheme

Based on the buffer system screening results, corresponding adjuvants were added to the F5 formulation, including glycine, sucrose, trehalose, and mannitol, a total of 4 formulations. The protein had a concentration of 10 mg/mL. All formulations used 0.04% polysorbate 80 as the surfactant. The samples were subjected to accelerated test at 40°C and freeze-thaw test, and the stability of each formulation was investigated with the appearance, pH value, concentration, osmotic pressure molar concentration, purity (molecular exclusion chromatography (SEC-HPLC), non-reduced sodium dodecyl sulfate capillary electrophoresis (CE-SDS(NR), reduced sodium dodecyl sulfate capillary electrophoresis (CE-SDS(R)), charge isomerism (full-column capillary isoelectric focusing electrophoresis (iCIEF)), dynamic light scattering (DLS) and binding activity (detected by ELISA ) as indicators, so as to screen the optimal adjuvant. The specific formulation composition and investigation scheme are shown in Table 7.

**Table 7 Adjuvant screening scheme**

| Formula tion | Formulation composition | | T0 | 40°C | | | -40°C to room temperature | |
|---|---|---|---|---|---|---|---|---|
| | | | | 1W | 2W | 4W | 3 rounds | 5 rounds |
| F5-1 | 20 mM histidine-histidine hydrochlori de, pH 6.0 | 2% glycine, 0.04% polysorbate 80 | X,Y | X | X | X,Y | X | X |
| F5-2 | | 8% sucrose, 0.04% polysorbate 80 | X,Y | X | X | X,Y | X | X |
| F5-3 | | 8% trehalose, 0.04% polysorbate 80 | X,Y | X | X | X,Y | X | X |
| F5-4 | | 4.5% mannitol, 0.04% polysorbate 80 | X,Y | X | X | X,Y | X | X |

| | | | | | | | | |
|---|---|---|---|---|---|---|---|---|
| Remark: X = Appearance, pH, concentration, iCIEF, SEC-HPLC, CE-SDS(NR), CE-SDS(R), DLS, and binding activity; Y = osmotic pressure molar concentration T0 = starting point of investigation; 1 W = 1 week, and so on. | | | | | | | | |

### 3.2 Adjuvant screening results

The main results of adjuvant screening are summarized in Table 8, Table 9, Table 10, Table 11, Table 12 and Table 13.

**Table 8 Result 1 of 40°C accelerated test for adjuvant screening**

| No. | Investigation conditions | Appearance | Protein concentratio n (mg/mL) | Particle size (nm) | pH | Osmotic pressure molar concentration (mOsmol/kg) |
|---|---|---|---|---|---|---|
| | T0 | Colorless, slight opalescence, no visible particles | 10.38 | 13.45 | 6.142 | 291 |
| F5-1 | 40°C, 1 week | Colorless, slight opalescence, no visible particles | 10.13 | 17.39 | 6.151 | N/A |
| | 40°C, 2 weeks | Colorless, slight opalescence, no visible particles | 10.10 | 20.99 | 6.149 | N/A |
| | 40°C, 4 weeks | Colorless, slight opalescence, no visible particles | 10.07 | 23.42 | 6.138 | 298 |
| F5-2 | T0 | Colorless, slight opalescence, no visible particles | 10.54 | 16.09 | 6.053 | 294 |
| | 40°C, 1 week | Colorless, slight opalescence, no visible particles | 10.42 | 19.64 | 6.084 | N/A |
| | 40°C, 2 weeks | Colorless, slight opalescence, no visible particles | 10.62 | 23.00 | 6.063 | N/A |
| | 40°C, 4 weeks | Colorless, slight opalescence, no visible particles | 10.81 | 26.10 | 6.090 | 302 |
| F5-3 | T0 | Colorless, slight opalescence, no visible particles | 10.50 | 15.65 | 6.066 | 269 |
| | 40°C, 1 week | Colorless, slight opalescence, no visible particles | 10.45 | 20.34 | 6.066 | N/A |
| | 40°C, 2 weeks | Colorless, slight opalescence, no visible particles | 10.48 | 22.74 | 6.067 | N/A |
| | 40°C, 4 weeks | Colorless, slight opalescence, no visible particles | 10.73 | 26.77 | 6.142 | 275 |
| F5-4 | T0 | Colorless, slight opalescence, no visible particles | 10.30 | 15.07 | 6.069 | 293 |
| | 40°C, 1 week | Colorless, slight opalescence, no visible particles | 10.45 | 19.53 | 6.088 | N/A |
| | 40°C, 2 weeks | Colorless, slight opalescence, no visible particles | 10.36 | 22.09 | 6.073 | N/A |
| | 40°C, 4 weeks | Colorless, slight opalescence, no visible particles | 10.12 | 25.73 | 6.088 | 300 |

| | | | | | | |
|---|---|---|---|---|---|---|
| Remark: T0 = starting point of investigation; N/A = not applicable | | | | | | |

**Table 9 Result 2 of 40°C accelerated test for adjuvant screening**

| No. | Investigation conditions | SEC-HPLC(%) | | | CE-SDS (NR) (%) | | |
|---|---|---|---|---|---|---|---|
| | | Low molecular weight peak | Main peak | High molecular weight peak | Low molecular weight peak | Main peak | High molecular weight peak |
| F5-1 | T0 | ND | 98.6 | 1.4 | 2.91 | 97.09 | 0.00 |
| | 40°C, 1 week | 1.2 | 96.2 | 2.7 | 5.20 | 94.80 | 0.00 |
| | 40°C, 2 weeks | 1.3 | 95.1 | 3.6 | 5.50 | 94.50 | 0.00 |
| | 40°C, 4 weeks | 2.1 | 94.4 | 3.5 | 9.31 | 90.69 | 0.00 |
| F5-2 | T0 | ND | 98.7 | 1.3 | 2.56 | 97.44 | 0.00 |
| | 40°C, 1 week | 1.2 | 96.3 | 2.5 | 4.20 | 95.80 | 0.00 |
| | 40°C, 2 weeks | 1.2 | 95.6 | 3.2 | 4.46 | 95.54 | 0.00 |
| | 40°C, 4 weeks | 1.8 | 95.1 | 3.1 | 8.24 | 91.76 | 0.00 |
| F5-3 | T0 | ND | 98.7 | 1.3 | 2.76 | 97.24 | 0.00 |
| | 40°C, 1 week | 1.3 | 96.0 | 2.7 | 4.51 | 95.49 | 0.00 |
| | 40°C, 2 weeks | 1.2 | 95.5 | 3.3 | 4.67 | 95.33 | 0.00 |
| | 40°C, 4 weeks | 1.8 | 95.1 | 3.2 | 8.47 | 91.53 | 0.00 |
| F5-4 | T0 | ND | 98.6 | 1.4 | 2.81 | 97.19 | 0.00 |
| | 40°C, 1 week | 1.3 | 95.8 | 2.9 | 4.92 | 95.08 | 0.00 |
| | 40°C, 2 weeks | 1.3 | 95.4 | 3.3 | 4.84 | 95.16 | 0.00 |
| | 40°C, 4 weeks | 2.2 | 94.4 | 3.4 | 8.39 | 91.61 | 0.00 |

| | | | | | | | |
|---|---|---|---|---|---|---|---|
| Remark: T0 = starting point of investigation; ND = not detected | | | | | | | |

**Table 10 Result 3 of 40°C accelerated test for adjuvant screening**

| No. | Investigation conditions | CE-SDS (R) (%) | | Activity % | iCIEF | | | |
|---|---|---|---|---|---|---|---|---|
| | | Main peak | Others | | Isoelec tric point | Acidic peak | Main peak | Basic peak |
| F5-1 | T0 | 99.69 | 0.31 | 95 | 8.489 | 32.9 | 60.4 | 6.7 |
| | 40°C, 1 week | 97.47 | 2.53 | 72 | 8.504 | 43.3 | 49.9 | 6.7 |
| | 40°C, 2 weeks | 97.08 | 2.92 | 58 | 8.664 | 53.3 | 40.1 | 6.6 |
| | 40°C, 4 weeks | 95.98 | 4.02 | 42 | 8.659 | 62.4 | 20.3 | 17.4 |
| F5-2 | T0 | 99.78 | 0.22 | 97 | 8.334 | 29.6 | 64.2 | 6.1 |
| | 40°C, 1 week | 97.67 | 2.33 | 79 | 8.335 | 41.1 | 52.2 | 6.7 |
| | 40°C, 2 weeks | 97.32 | 2.68 | 66 | 8.480 | 48.7 | 43.9 | 7.3 |
| | 40°C, 4 weeks | 96.40 | 3.60 | 46 | 8.473 | 52.7 | 27.4 | 19.8 |
| F5-3 | T0 | 99.74 | 0.26 | 105 | 8.350 | 30.3 | 63.1 | 6.7 |
| | 40°C, 1 week | 97.77 | 2.23 | 80 | 8.344 | 40.8 | 52.4 | 6.8 |
| | 40°C, 2 weeks | 97.37 | 2.63 | 65 | 8.500 | 51.2 | 41.5 | 7.3 |
| | 40°C, 4 weeks | 96.29 | 3.71 | 47 | 8.498 | 54.6 | 26.9 | 18.4 |
| F5-4 | T0 | 99.73 | 0.27 | 103 | 8.346 | 29.9 | 64.1 | 6.0 |
| | 40°C, 1 week | 97.90 | 2.10 | 76 | 8.343 | 40.8 | 52.8 | 6.4 |
| | 40°C, 2 weeks | 97.33 | 2.67 | 68 | 8.499 | 49.2 | 44.4 | 6.3 |
| | 40°C, 4 weeks | 96.01 | 3.99 | 51 | 8.498 | 59.6 | 22.7 | 17.6 |

| | | | | | | | | |
|---|---|---|---|---|---|---|---|---|
| Remark: T0 = starting point of investigation | | | | | | | | |

**Table 11 Result 1 of freeze-thaw test for adjuvant screening**

| No. | Investigation conditions | Appearance | Protein concentration (mg/mL) | Particle size (nm) | pH | Osmotic pressure molar concentration (mOsmol/kg) |
|---|---|---|---|---|---|---|
| F5-1 | T0 | Colorless, slight opalescence, no visible particles | 10.38 | 13.45 | 6.142 | 291 |
| | 3 rounds of freeze-thaw from -40°C to room temperature | Colorless, slight opalescence, no visible particles | 10.32 | 13.66 | 6.138 | N/A |
| | 5 rounds of freeze-thaw from -40°C to room temperature | Colorless, slight opalescence, no visible particles | 10.29 | 13.24 | 6.088 | N/A |
| F5-2 | T0 | Colorless, slight opalescence, no visible particles | 10.54 | 16.09 | 6.053 | 294 |
| | 3 rounds of freeze-thaw from -40°C to room temperature | Colorless, slight opalescence, no visible particles | 10.45 | 16.31 | 6.089 | N/A |
| | 5 rounds of freeze-thaw from -40°C to room temperature | Colorless, slight opalescence, no visible particles | 10.51 | 15.48 | 6.040 | N/A |
| F5-3 | T0 | Colorless, slight opalescence, no visible particles | 10.50 | 15.65 | 6.066 | 269 |
| | 3 rounds of freeze-thaw from -40°C to room temperature | Colorless, slight opalescence, no visible particles | 10.42 | 16.02 | 6.091 | N/A |
| | 5 rounds of freeze-thaw from -40°C to room temperature | Colorless, slight opalescence, no visible particles | 10.51 | 15.99 | 6.031 | N/A |
| F5-4 | T0 | Colorless, slight opalescence, no visible particles | 10.30 | 15.07 | 6.069 | 293 |
| | 3 rounds of freeze-thaw from -40°C to room temperature | Colorless, slight opalescence, no visible particles | 10.30 | 14.34 | 6.093 | N/A |
| | 5 rounds of freeze-thaw from -40°C to room temperature | Colorless, slight opalescence, no visible particles | 10.39 | 15.57 | 6.028 | N/A |

| | | | | | | |
|---|---|---|---|---|---|---|
| Remark: T0 = starting point of investigation; N/A = not applicable | | | | | | |

**Table 12 Result 2 of freeze-thaw test for adjuvant screening**

| No. | Investigation conditions | SEC-HPLC(%) | | | CE-SDS (NR) (%) | | |
|---|---|---|---|---|---|---|---|
| | | Low molecular weight peak | Main peak | High molecular weight peak | Low molecular weight peak | Main peak | High molecular weight peak |
| F5-1 | T0 | ND | 98.6 | 1.4 | 2.91 | 97.09 | 0.00 |
| | 3 rounds of freeze-thaw from -40°C to room temperature | 0.1 | 97.9 | 2.0 | 2.55 | 97.45 | 0.00 |
| | 5 rounds of freeze-thaw from -40°C to room temperature | 0.7 | 96.9 | 2.4 | 1.78 | 98.22 | 0.00 |
| F5-2 | T0 | ND | 98.7 | 1.3 | 2.56 | 97.44 | 0.00 |
| | 3 rounds of freeze-thaw from -40°C to room temperature | 0.1 | 98.6 | 1.3 | 2.61 | 97.39 | 0.00 |
| | 5 rounds of freeze-thaw from -40°C to room temperature | 0.6 | 98.1 | 1.3 | 1.73 | 98.27 | 0.00 |
| F5-3 | T0 | ND | 98.7 | 1.3 | 2.76 | 97.24 | 0.00 |
| | 3 rounds of freeze-thaw from -40°C to room temperature | 0.1 | 98.5 | 1.3 | 2.78 | 97.22 | 0.00 |
| | 5 rounds of freeze-thaw from -40°C to room temperature | 0.6 | 98.1 | 1.3 | 1.80 | 98.20 | 0.00 |
| F5-4 | T0 | ND | 98.6 | 1.4 | 2.81 | 97.19 | 0.00 |
| | 3 rounds of freeze-thaw from -40°C to room temperature | 0.1 | 96.9 | 3.0 | 2.55 | 97.45 | 0.00 |
| | 5 rounds of freeze-thaw from -40°C to room temperature | 0.7 | 95.6 | 3.7 | 1.77 | 98.23 | 0.00 |

| | | | | | | | |
|---|---|---|---|---|---|---|---|
| Remark: T0 = starting point of investigation; ND = not detected | | | | | | | |

**Table 13 Result 3 of freeze-thaw test for adjuvant screening**

| No. | Investigation conditions | CE-SDS (R) (%) | | Activity% | iCIEF | | | |
|---|---|---|---|---|---|---|---|---|
| | | Main peak | Others | | Isoelectric point | Acidic peak | Main peak | Basic peak |
| F5-1 | T0 | 99.69 | 0.31 | 95 | 8.489 | 32.9 | 60.4 | 6.7 |
| | 3 rounds of freeze-thaw from -40°C to room temperature | 99.54 | 0.46 | 101 | 8.501 | 34.4 | 59.3 | 6.4 |
| | 5 rounds of freeze-thaw from -40°C to room temperature | 99.81 | 0.19 | 97 | 8.636 | 33.9 | 59.7 | 6.5 |
| F5-2 | T0 | 99.78 | 0.22 | 97 | 8.334 | 29.6 | 64.2 | 6.1 |
| | 3 rounds of freeze-thaw from -40°C to room temperature | 99.74 | 0.26 | 99 | 8.337 | 32.7 | 61.0 | 6.4 |
| | 5 rounds of freeze-thaw from -40°C to room temperature | 99.74 | 0.26 | 95 | 8.486 | 32.4 | 61.0 | 6.7 |
| F5-3 | T0 | 99.74 | 0.26 | 105 | 8.350 | 30.3 | 63.1 | 6.7 |
| | 3 rounds of freeze-thaw from -40°C to room temperature | 99.73 | 0.27 | 106 | 8.346 | 30.6 | 63.0 | 6.4 |
| | 5 rounds of freeze-thaw from -40°C to room temperature | 99.68 | 0.32 | 96 | 8.506 | 32.3 | 61.3 | 6.4 |
| F5-4 | T0 | 99.73 | 0.27 | 103 | 8.346 | 29.9 | 64.1 | 6.0 |
| | 3 rounds of freeze-thaw from -40°C to room temperature | 99.67 | 0.33 | 101 | 8.345 | 32.6 | 61.0 | 6.5 |
| | 5 rounds of freeze-thaw from -40°C to room temperature | 99.67 | 0.33 | 92 | 8.517 | 34.2 | 59.1 | 6.7 |

| | | | | | | | | |
|---|---|---|---|---|---|---|---|---|
| Remark: T0 = starting point of investigation | | | | | | | | |

### 3.3 Conclusion of adjuvant screening

The samples had a concentration of 10 mg/mL, and were subjected to 40°C for 4 weeks and 5 rounds of freeze-thaw tests from -40°C to room temperature to screen adjuvants. The appearance of all formulations was colorless and slightly opalescent liquid with no visible particles. The pH was within the target value and there was no significant change in concentration.
(1) Accelerated investigation at 40°C for 4 weeks.
   **SEC-HPLC purity:** There were no significant differences among the formulations.
   **CE-SDS (NR):** F5-2, F5-3 and F5-4 > F5-1, the results showed that the purity of the glycine-containing formulation was slightly lower than other formulations.
   **CE-SDS (R):** There were no significant differences among the formulations.
   **iCIEF:** The degree of change in the acidic peaks of F5-2 and F5-3 was slightly lower than that of other formulations, but the changes in the acidic and alkaline peaks did not lead to differences in binding activity.
   **Binding activity:** All dropped to between 40% and 60%, with no significant difference among the formulations.
(2) 5 rounds of freeze-thaw from -40°C to room temperature.
   **SEC-HPLC:** F5-2 and F5-3 > F5-1 and F5-4, the main peak purity of the formulations containing sucrose and trehalose were slightly higher than those of the formulations containing glycine and mannitol.
   **CE-SDS(NR), CE-SDS(R), iCIEF and binding activity:** There were no significant differences among the formulations.

In summary, the formulations F5-3 (containing trehalose) and F5-2 (containing sucrose) were better than other formulations in the adjuvant screening process.

Since the binding activity in the liquid state had dropped to between 40% and 60%, the development of a lyophilized preparation would be carried out.

In the lyophilized powder state, a differential scanning calorimeter (DSC) was used to detect the glass transition temperature (Tg) of the sample. Under the condition of programmed temperature control, the temperature was raised from 0°C to 150°C, and the glass transition temperature of the lyophilized powder sample was detected by measuring the relationship between the heat flux difference input to the sample and the reference and the temperature during the temperature rise process. Generally, the higher the glass transition temperature of a substance, the better the stability of the substance at high temperature. Wherein, the glass transition temperature (Tg) of F5-3 (containing trehalose) was 115.86°C, the glass transition temperature (Tg) of F5-2 (containing sucrose) was 56.97°C, and the glass transition temperature of F5-3 was obviously higher than that of F5-2 formulation, indicating that trehalose in a reasonable concentration range could improve the stability of the preparation. Therefore, trehalose was determined as the optimal adjuvant, and trehalose was used for the next surfactant screening.

### Example 4. Surfactant screening

### 4.1 Surfactant screening scheme

Different concentrations of surfactants were added to the screened buffer system and adjuvant. There was a total of 8 formulations including 0.02%, 0.04%, 0.06%, and 0.08% polysorbate 80 and poloxamer 188, respectively. The protein concentration of each formulation was 10 mg/mL. Through accelerated test at 40°C and shaking at 300 rpm and 25°C, the stability of each formulation was investigated with the appearance, pH value, concentration, surfactant concentration, purity (molecular exclusion chromatography (SEC-HPLC), non-reduced sodium dodecyl sulfate capillary electrophoresis (CE-SDS(NR), reduced sodium dodecyl sulfate capillary electrophoresis (CE-SDS(R)), charge isomerism (full-column capillary isoelectric focusing electrophoresis (iCIEF)), dynamic light scattering (DLS), insoluble microparticles and binding activity as indicators, so as to screen the optimal surfactant. The specific formulation composition and investigation scheme are shown in Table 14.

**Table 14 Surfactant screening scheme**

| Formulation | Formulation composition | | T0 | 40°C | | | 300 rpm 25°C | |
|---|---|---|---|---|---|---|---|---|
| | | | | 1W | 2W | 4W | 3D | 7D |
| F5-3-1 | | 0.02% polysorbate 80 | X,Y | X | X | X,Y | X | X,Y |
| F5-3-2 | | 0.04% polysorbate 80 | X,Y | X | X | X,Y | X | X,Y |
| F5-3-3 | 20 mM Histidine - histidine hydrochlori de, 8% Trehalose, pH 6.0 | 0.06% polysorbate 80 | X,Y | X | X | X,Y | X | X,Y |
| F5-3-4 | | 0.08% polysorbate 80 | X,Y | X | X | X,Y | X | X,Y |
| F5-3-5 | | 0.02% poloxamer 188 | X,Y | X | X | X,Y | X | X,Y |
| F5-3-6 | | 0.04% poloxamer 188 | X,Y | X | X | X,Y | X | X,Y |
| F5-3-7 | | 0.06% poloxamer 188 | X,Y | X | X | X,Y | X | X,Y |
| F5-3-8 | | 0.08% poloxamer 188 | X,Y | X | X | X,Y | X | X,Y |

| | | | | | | | | |
|---|---|---|---|---|---|---|---|---|
| Remark: X = Appearance, pH, concentration, surfactant concentration, iCIEF, SEC-HPLC, CE-SDS(NR), CE-SDS(R), DLS, and activity; Y = insoluble microparticles T0 = starting point of investigation; 1 W = 1 week; 1 D = 1 day, and so on. | | | | | | | | |

### 4.2 Surfactant screening results

The main results of surfactant screening are summarized in Table 15, Table 16, Table 17, Table 18, Table 19 and Table 20.

**Table 15 Result 1 of 40°C accelerated test for surfactant screening**

| No. | Investigation conditions | Appearance | Protein concentration (mg/mL) | Particle size (nm) | pH | Insoluble microparticles (particles/mL) | |
|---|---|---|---|---|---|---|---|
| | | | | | | ≥ 10 µm | ≥ 25 µm |
| F5-3-1 | T0 | Colorless, slight opalescence, no visible particles | 10.17 | 16.50 | 6.092 | 0.00 | 0.00 |
| | 40°C, 1 week | Colorless, slight opalescence, no visible particles | 10.02 | 21.47 | 6.103 | N/A | N/A |
| | 40°C, 2 weeks | Colorless, slight opalescence, no visible particles | 10.28 | 24.79 | 6.088 | N/A | N/A |
| | 40°C, 4 weeks | Colorless, slight opalescence, no visible particles | 10.19 | 31.27 | 6.115 | 54.46 | 4.54 |
| F5-3-2 | T0 | Colorless, slight opalescence, no visible particles | 10.27 | 16.38 | 6.087 | 0.00 | 0.00 |
| | 40°C, 1 week | Colorless, slight opalescence, no visible particles | 9.99 | 21.56 | 6.091 | N/A | N/A |
| | 40°C, 2 weeks | Colorless, slight opalescence, no visible particles | 10.15 | 24.62 | 6.064 | N/A | N/A |
| | 40°C, 4 weeks | Colorless, slight opalescence, no visible particles | 9.93 | 31.47 | 6.118 | 2.27 | 0.00 |
| F5-3-3 | T0 | Colorless, slight opalescence, no visible particles | 10.32 | 16.39 | 6.077 | 2.27 | 0.00 |
| | 40°C, 1 week | Colorless, slight opalescence, no visible particles | 9.89 | 22.23 | 6.090 | N/A | N/A |
| | 40°C, 2 weeks | Colorless, slight opalescence, no visible particles | 10.18 | 25.31 | 6.073 | N/A | N/A |
| | 40°C, 4 weeks | Colorless, slight opalescence, no visible particles | 9.97 | 30.84 | 6.122 | 24.96 | 13.61 |
| F5-3-4 | T0 | Colorless, slight opalescence, no visible particles | 10.20 | 17.37 | 6.080 | 4.54 | 0.00 |
| | 40°C, 1 week | Colorless, slight opalescence, no visible particles | 10.00 | 22.65 | 6.076 | N/A | N/A |
| | 40°C, 2 weeks | Colorless, slight opalescence, no visible particles | 10.10 | 26.23 | 6.080 | N/A | N/A |
| | 40°C, 4 weeks | Colorless, slight opalescence, no visible particles | 10.02 | 32.11 | 6.114 | 27.23 | 9.08 |
| F5-3-5 | T0 | Colorless, slight opalescence, no visible particles | 9.92 | 16.58 | 6.104 | 9.08 | 0.00 |
| | 40°C, 1 week | Colorless, slight opalescence, no visible particles | 9.99 | 17.88 | 6.105 | N/A | N/A |
| | 40°C, 2 weeks | Colorless, slight opalescence, no visible particles | 10.15 | 21.25 | 6.074 | N/A | N/A |
| | 40°C, 4 weeks | Colorless, slight opalescence, no visible particles | 9.92 | 26.27 | 6.107 | 15.88 | 0.00 |
| F5-3-6 | T0 | Colorless, slight opalescence, no visible particles | 9.94 | 16.38 | 6.096 | 4.54 | 0.00 |
| | 40°C, 1 week | Colorless, slight opalescence, no visible particles | 9.93 | 17.50 | 6.082 | N/A | N/A |
| | 40°C, 2 weeks | Colorless, slight opalescence, no visible particles | 10.09 | 23.24 | 6.085 | N/A | N/A |
| | 40°C, 4 weeks | Colorless, slight opalescence, no visible particles | 9.92 | 25.85 | 6.112 | 2.27 | 0.00 |
| F5-3-7 | T0 | Colorless, slight opalescence, no visible particles | 9.97 | 16.47 | 6.099 | 34.40 | 6.81 |
| | 40°C, 1 week | Colorless, slight opalescence, no visible particles | 9.98 | 17.61 | 6.107 | N/A | N/A |
| | 40°C, 2 weeks | Colorless, slight opalescence, no visible particles | 10.11 | 20.61 | 6.090 | N/A | N/A |
| | 40°C, 4 weeks | Colorless, slight opalescence, no visible particles | 9.90 | 25.94 | 6.096 | 29.50 | 4.54 |
| F5-3-8 | T0 | Colorless, slight opalescence, no visible particles | 10.03 | 16.94 | 6.057 | 2.27 | 0.00 |
| | 40°C, 1 week | Colorless, slight opalescence, no visible particles | 9.94 | 18.00 | 6.085 | N/A | N/A |
| | 40°C, 2 weeks | Colorless, slight opalescence, no visible particles | 10.10 | 20.76 | 6.057 | N/A | N/A |
| | 40°C, 4 weeks | Colorless, slight opalescence, no visible particles | 9.91 | 27.32 | 6.111 | 47.65 | 18.15 |

| | | | | | | | |
|---|---|---|---|---|---|---|---|
| Remark: T0 = starting point of investigation; N/A = not applicable | | | | | | | |

**Table 16 Result 2 of 40°C accelerated test for surfactant screening**

| No. | Investigation conditions | SEC-HPLC(%) | | | Activity % | CE-SDS (NR) (%) | | |
|---|---|---|---|---|---|---|---|---|
| | | Low molecular weight peak | Main peak | High molecular weight peak | | Low molecular weight peak | Main peak | High molecular weight peak |
| F5-3-1 | T0 | ND | 98.4 | 1.6 | 97 | 1.56 | 98.44 | 0.00 |
| | 40°C, 1 week | 0.7 | 94.5 | 4.9 | 82 | 3.01 | 96.99 | ND |
| | 40°C, 2 weeks | 1.1 | 93.6 | 5.4 | 60 | 4.70 | 95.30 | 0.00 |
| | 40°C, 4 weeks | 1.4 | 97.7 | 1.0 | 40 | 9.31 | 90.38 | 0.32 |
| F5-3-2 | T0 | ND | 98.4 | 1.6 | 97 | 1.63 | 97.82 | 0.55 |
| | 40°C, 1 week | 0.7 | 94.2 | 5.1 | 82 | 3.62 | 95.83 | 0.54 |
| | 40°C, 2 weeks | 1.1 | 93.5 | 5.4 | 62 | 5.09 | 93.67 | 1.25 |
| | 40°C, 4 weeks | 1.4 | 97.4 | 1.2 | 43 | 8.50 | 90.13 | 1.38 |
| F5-3-3 | T0 | ND | 98.4 | 1.6 | 97 | 1.52 | 98.48 | 0.00 |
| | 40°C, 1 week | 0.9 | 95.3 | 3.8 | 79 | 2.19 | 97.81 | ND |
| | 40°C, 2 weeks | 1.1 | 93.6 | 5.3 | 64 | 4.93 | 95.07 | 0.00 |
| | 40°C, 4 weeks | 1.3 | 97.5 | 1.2 | 47 | 8.68 | 91.07 | 0.25 |
| F5-3-4 | T0 | ND | 98.4 | 1.6 | 102 | 1.43 | 98.57 | 0.00 |
| | 40°C, 1 week | 0.6 | 94.2 | 5.2 | 79 | 2.77 | 97.23 | ND |
| | 40°C, 2 weeks | 1.1 | 93.5 | 5.3 | 59 | 5.25 | 94.75 | 0.00 |
| | 40°C, 4 weeks | 1.3 | 97.3 | 1.4 | 45 | 8.92 | 90.82 | 0.27 |
| F5-3-5 | T0 | ND | 98.4 | 1.6 | 99 | 1.41 | 98.59 | 0.00 |
| | 40°C, 1 week | 0.6 | 94.1 | 5.3 | 83 | 2.74 | 97.26 | ND |
| | 40°C, 2 weeks | 0.9 | 93.4 | 5.7 | 69 | 4.54 | 95.46 | 0.00 |
| | 40°C, 4 weeks | 1.0 | 96.9 | 2.1 | 47 | 8.67 | 90.92 | 0.41 |
| F5-3-6 | T0 | ND | 98.3 | 1.7 | 97 | 1.80 | 98.20 | 0.00 |
| | 40°C, 1 week | 0.6 | 94.1 | 5.4 | 87 | 2.79 | 97.21 | ND |
| | 40°C, 2 weeks | 1.0 | 92.4 | 6.6 | 63 | 6.37 | 93.63 | 0.00 |
| | 40°C, 4 weeks | 1.2 | 96.8 | 2.0 | 51 | 8.78 | 90.98 | 0.24 |
| F5-3-7 | T0 | ND | 98.4 | 1.6 | 100 | 1.51 | 98.49 | 0.00 |
| | 40°C, 1 week | 0.6 | 94.0 | 5.4 | 85 | 2.68 | 97.32 | ND |
| | 40°C, 2 weeks | 1.0 | 93.4 | 5.7 | 64 | 4.72 | 95.28 | 0.00 |
| | 40°C, 4 weeks | 1.0 | 96.7 | 2.3 | 50 | 8.33 | 91.44 | 0.23 |
| F5-3-8 | T0 | ND | 98.4 | 1.6 | 98 | 1.23 | 98.77 | 0.00 |
| | 40°C, 1 week | 0.6 | 94.0 | 5.4 | 83 | 3.01 | 96.99 | ND |
| | 40°C, 2 weeks | 1.0 | 93.3 | 5.7 | 66 | 4.65 | 95.35 | 0.00 |
| | 40°C, 4 weeks | 1.0 | 96.4 | 2.6 | 50 | 8.93 | 90.86 | 0.21 |

| | | | | | | | | |
|---|---|---|---|---|---|---|---|---|
| Remark: T0 = starting point of investigation; ND = not detected | | | | | | | | |

**Table 17 Result 3 of 40°C accelerated test for surfactant screening**

| No. | Investigation conditions | CE-SDS (R) (%) | | iCIEF | | | | Surfactant concentration (mg/ml) | |
|---|---|---|---|---|---|---|---|---|---|
| | | Main peak | Others | Isoelectric point | Acidic peak | Main peak | Basic peak | Theoretical concentratio n | Detected concentration |
| F5-3-1 | T0 | 99.68 | 0.32 | 8.496 | 38.4 | 55.1 | 6.5 | 0.2 | 0.19 |
| | 40°C, 1 week | 97.69 | 2.31 | 8.493 | 46.4 | 46.2 | 7.3 | 0.2 | 0.19 |
| | 40°C, 2 weeks | 96.10 | 3.90 | 8.466 | 48.5 | 45.6 | 5.9 | 0.2 | 0.18 |
| | 40°C, 4 weeks | 95.54 | 4.46 | 8.503 | 62.2 | 22.3 | 15.6 | 0.2 | 0.18 |
| F5-3-2 | T0 | 99.44 | ND | 8.497 | 40.1 | 53.2 | 6.9 | 0.4 | 0.41 |
| | 40°C, 1 week | 97.78 | 1.45 | 8.496 | 45.9 | 47.2 | 7.0 | 0.4 | 0.41 |
| | 40°C, 2 weeks | 96.80 | 2.36 | 8.478 | 50.7 | 43.1 | 6.1 | 0.4 | 0.39 |
| | 40°C, 4 weeks | 94.85 | 4.09 | 8.504 | 63.2 | 21.3 | 15.5 | 0.4 | 0.39 |
| F5-3-3 | T0 | 99.60 | 0.40 | 8.496 | 38.5 | 54.7 | 6.8 | 0.6 | 0.59 |
| | 40°C, 1 week | 97.78 | 2.22 | 8.493 | 46.4 | 47.1 | 6.4 | 0.6 | 0.60 |
| | 40°C, 2 weeks | 96.25 | 3.75 | 8.465 | 50.7 | 43.1 | 6.2 | 0.6 | 0.59 |
| | 40°C, 4 weeks | 95.39 | 4.61 | 8.501 | 58.6 | 24.8 | 16.7 | 0.6 | 0.57 |
| F5-3-4 | T0 | 99.67 | 0.33 | 8.497 | 37.8 | 55.4 | 6.7 | 0.8 | 0.78 |
| | 40°C, 1 week | 97.56 | 2.44 | 8.496 | 46.1 | 47.5 | 6.3 | 0.8 | 0.80 |
| | 40°C, 2 weeks | 96.72 | 3.28 | 8.464 | 53.7 | 40.6 | 5.6 | 0.8 | 0.77 |
| | 40°C, 4 weeks | 95.88 | 4.12 | 8.505 | 61.8 | 21.5 | 16.7 | 0.8 | 0.77 |
| F5-3-5 | T0 | 98.81 | 1.19 | 8.497 | 40.0 | 53.3 | 6.6 | 0.2 | 0.25 |
| | 40°C, 1 week | 97.76 | 2.24 | 8.493 | 42.7 | 50.2 | 7.2 | 0.2 | 0.27 |
| | 40°C, 2 weeks | 96.72 | 3.28 | 8.467 | 47.2 | 46.3 | 6.6 | 0.2 | 0.22 |
| | 40°C, 4 weeks | 95.53 | 4.47 | 8.504 | 55.5 | 23.8 | 20.7 | 0.2 | 0.25 |
| F5-3-6 | T0 | 98.28 | 1.72 | 8.497 | 39.9 | 53.4 | 6.8 | 0.4 | 0.38 |
| | 40°C, 1 week | 97.97 | 2.03 | 8.497 | 40.9 | 51.8 | 7.3 | 0.4 | 0.39 |
| | 40°C, 2 weeks | 96.40 | 3.60 | 8.465 | 50.8 | 43.3 | 5.9 | 0.4 | 0.34 |
| | 40°C, 4 weeks | 95.63 | 4.37 | 8.504 | 55.2 | 25.5 | 19.3 | 0.4 | 0.37 |
| F5-3-7 | T0 | 96.63 | 3.37 | 8.508 | 38.0 | 55.5 | 6.5 | 0.6 | 0.59 |
| | 40°C, 1 week | 97.74 | 2.26 | 8.494 | 43.2 | 49.8 | 6.9 | 0.6 | 0.64 |
| | 40°C, 2 weeks | 97.15 | 2.85 | 8.466 | 46.9 | 45.6 | 7.4 | 0.6 | 0.54 |
| | 40°C, 4 weeks | 95.52 | 4.48 | 8.503 | 54.2 | 25.2 | 20.6 | 0.6 | 0.58 |
| F5-3-8 | T0 | 96.07 | 3.93 | 8.496 | 39.6 | 53.8 | 6.7 | 0.8 | 0.84 |
| | 40°C, 1 week | 97.74 | 2.26 | 8.493 | 42.6 | 50.4 | 6.9 | 0.8 | 0.91 |
| | 40°C, 2 weeks | 96.30 | 3.70 | 8.466 | 47.1 | 45.8 | 7.2 | 0.8 | 0.82 |
| | 40°C, 4 weeks | 94.94 | 5.06 | 8.504 | 53.8 | 25.9 | 20.2 | 0.8 | 0.83 |

| | | | | | | | | | |
|---|---|---|---|---|---|---|---|---|---|
| Remark: T0 = starting point of investigation | | | | | | | | | |

**Table 18 Result 1 of shaking test for surfactant screening**

| No. | Investigation conditions | Appearance | Protein concentration (mg/mL) | Particle size (nm) | pH | Insoluble microparticles (particles/mL) | |
|---|---|---|---|---|---|---|---|
| | | | | | | ≥ 10 µm | ≥ 25 µm |
| F5-3-1 | T0 | Colorless, slight opalescence, no visible particles | 10.17 | 16.50 | 6.092 | 0.00 | 0.00 |
| | Shaking at 25°C, 300 rpm for 3 days | Colorless, slight opalescence, no visible particles | 10.19 | 17.20 | 6.153 | N/A | N/A |
| | Shaking at 25°C, 300 rpm for 7 days | Colorless, slight opalescence, no visible particles | 9.99 | 16.45 | 6.105 | 0.00 | 0.00 |
| F5-3-2 | T0 | Colorless, slight opalescence, no visible particles | 10.27 | 16.38 | 6.087 | 0.00 | 0.00 |
| | Shaking at 25°C, 300 rpm for 3 days | Colorless, slight opalescence, no visible particles | 10.11 | 16.80 | 6.131 | N/A | N/A |
| | Shaking at 25°C, 300 rpm for 7 days | Colorless, slight opalescence, no visible particles | 10.08 | 16.10 | 6.052 | 0.00 | 0.00 |
| F5-3-3 | T0 | Colorless, slight opalescence, no visible particles | 10.32 | 16.39 | 6.077 | 2.27 | 0.00 |
| | Shaking at 25°C, 300 rpm for 3 days | Colorless, slight opalescence, no visible particles | 9.94 | 16.57 | 6.114 | N/A | N/A |
| | Shaking at 25°C, 300 rpm for 7 days | Colorless, slight opalescence, no visible particles | 10.07 | 15.85 | 6.110 | 0.00 | 0.00 |
| F5-3-4 | T0 | Colorless, slight opalescence, no visible particles | 10.20 | 17.37 | 6.080 | 4.54 | 0.00 |
| | Shaking at 25°C, 300 rpm for 3 days | Colorless, slight opalescence, no visible particles | 10.12 | 16.85 | 6.137 | N/A | N/A |
| | Shaking at 25°C, 300 rpm for 7 days | Colorless, slight opalescence, no visible particles | 10.10 | 15.92 | 6.091 | 0.00 | 0.00 |
| F5-3-5 | T0 | Colorless, slight opalescence, no visible particles | 9.92 | 16.58 | 6.104 | 9.08 | 0.00 |
| | Shaking at 25°C, 300 rpm for 3 days | Colorless, slight opalescence, no visible particles | 10.03 | 16.69 | 6.122 | N/A | N/A |
| | Shaking at 25°C, 300 rpm for 7 days | Colorless, slight opalescence, no visible particles | 10.09 | 17.64 | 6.094 | 0.00 | 0.00 |
| F5-3-6 | T0 | Colorless, slight opalescence, no visible particles | 9.94 | 16.38 | 6.096 | 4.54 | 0.00 |
| | Shaking at 25°C, 300 rpm for 3 days | Colorless, slight opalescence, no visible particles | 10.04 | 16.74 | 6.104 | N/A | N/A |
| | Shaking at 25°C, 300 rpm for 7 days | Colorless, slight opalescence, no visible particles | 9.86 | 16.18 | 6.093 | 0.00 | 0.00 |
| F5-3-7 | T0 | Colorless, slight opalescence, no visible particles | 9.97 | 16.47 | 6.099 | 34.40 | 6.81 |
| | Shaking at 25°C, 300 rpm for 3 days | Colorless, slight opalescence, no visible particles | 9.87 | 16.72 | 6.107 | N/A | N/A |
| | Shaking at 25°C, 300 rpm for 7 days | Colorless, slight opalescence, no visible particles | 9.88 | 15.92 | 6.083 | 0.00 | 0.00 |
| F5-3-8 | T0 | Colorless, slight opalescence, no visible particles | 10.03 | 16.94 | 6.057 | 2.27 | 0.00 |
| | Shaking at 25°C, 300 rpm for 3 days | Colorless, slight opalescence, no visible particles | 9.92 | 17.57 | 6.124 | N/A | N/A |
| | Shaking at 25°C, 300 rpm for 7 days | Colorless, slight opalescence, visible feathery particles | 10.00 | 16.07 | 6.115 | 0.00 | 0.00 |

| | | | | | | | |
|---|---|---|---|---|---|---|---|
| Remark: T0 = starting point of investigation; N/A = not applicable | | | | | | | |

**Table 19 Result 2 of shaking test for surfactant screening**

| No. | Investigation conditions | SEC-HPLC(%) | | | Activity% | CE-SDS (NR) (%) | | |
|---|---|---|---|---|---|---|---|---|
| | | Low molecular weight peak | Main peak | High molecular weight peak | | Low molecular weight peak | Main peak | High molecular weight peak |
| F5-3-1 | T0 | ND | 98.4 | 1.6 | 97 | 1.56 | 98.44 | 0.00 |
| | Shaking at 25°C, 300 rpm for 3 days | 0.4 | 97.6 | 2.0 | 95 | 1.03 | 98.97 | ND |
| | Shaking at 25°C, 300 rpm for 7 days | 0.4 | 97.5 | 2.1 | 99 | 1.06 | 98.94 | ND |
| F5-3-2 | T0 | ND | 98.4 | 1.6 | 97 | 1.63 | 97.82 | 0.55 |
| | Shaking at 25°C, 300 rpm for 3 days | 0.4 | 97.5 | 2.0 | 103 | 1.30 | 98.24 | 0.45 |
| | Shaking at 25°C, 300 rpm for 7 days | 0.3 | 97.6 | 2.1 | 100 | 1.65 | 97.78 | 0.58 |
| F5-3-3 | T0 | ND | 98.4 | 1.6 | 97 | 1.52 | 98.48 | 0.00 |
| | Shaking at 25°C, 300 rpm for 3 days | 0.4 | 97.6 | 2.0 | 100 | 1.32 | 98.68 | ND |
| | Shaking at 25°C, 300 rpm for 7 days | 0.4 | 97.5 | 2.1 | 103 | 1.44 | 98.56 | ND |
| F5-3-4 | T0 | ND | 98.4 | 1.6 | 102 | 1.43 | 98.57 | 0.00 |
| | Shaking at 25°C, 300 rpm for 3 days | 0.4 | 97.6 | 2.0 | 103 | 1.46 | 98.54 | ND |
| | Shaking at 25°C, 300 rpm for 7 days | 0.4 | 97.5 | 2.1 | 100 | 1.14 | 98.86 | ND |
| F5-3-5 | T0 | ND | 98.4 | 1.6 | 99 | 1.41 | 98.59 | 0.00 |
| | Shaking at 25°C, 300 rpm for 3 days | 0.4 | 97.5 | 2.1 | 106 | 1.14 | 98.86 | ND |
| | Shaking at 25°C, 300 rpm for 7 days | 0.4 | 97.6 | 2.0 | 106 | 1.35 | 98.65 | ND |
| F5-3-6 | T0 | ND | 98.3 | 1.7 | 97 | 1.80 | 98.20 | 0.00 |
| | Shaking at 25°C, 300 rpm for 3 days | 0.4 | 97.5 | 2.1 | 100 | 1.31 | 98.69 | ND |
| | Shaking at 25°C, 300 rpm for 7 days | 0.4 | 97.6 | 2.1 | 107 | 1.45 | 98.55 | ND |
| F5-3-7 | T0 | ND | 98.4 | 1.6 | 100 | 1.51 | 98.49 | 0.00 |
| | Shaking at 25°C, 300 rpm for 3 days | 0.4 | 97.4 | 2.1 | 110 | 1.49 | 98.51 | ND |
| | Shaking at 25°C, 300 rpm for 7 days | 0.5 | 97.4 | 2.1 | 109 | 1.30 | 98.70 | ND |
| F5-3-8 | T0 | ND | 98.4 | 1.6 | 98 | 1.23 | 98.77 | 0.00 |
| | Shaking at 25°C, 300 rpm for 3 days | 0.4 | 97.6 | 2.0 | 107 | 1.30 | 98.70 | ND |
| | Shaking at 25°C, 300 rpm for 7 days | 0.4 | 97.5 | 2.1 | 107 | 1.30 | 98.70 | ND |

| | | | | | | | | |
|---|---|---|---|---|---|---|---|---|
| Remark: T0 = starting point of investigation; ND = not detected | | | | | | | | |

**Table 20 Result 3 of shaking test for surfactant screening**

| No. | Investigation conditions | CE-SDS (R) (%) | | iCIEF | | | | Surfactant concentration (mg/ml) | |
|---|---|---|---|---|---|---|---|---|---|
| | | Main peak | Others | Isoelectric point | Acidic peak | Main peak | Basic peak | Theoretical concentration | Actual concentration |
| F5-3-1 | T0 | 99.68 | 0.32 | 8.496 | 38.4 | 55.1 | 6.5 | 0.20 | 0.19 |
| | Shaking at 25°C, 300 rpm for 3 days | 99.47 | 0.53 | 8.499 | 38.3 | 55.3 | 6.2 | 0.20 | 0.20 |
| | Shaking at 25°C, 300 rpm for 7 days | 99.37 | 0.63 | 8.499 | 38.9 | 54.3 | 6.8 | 0.20 | 0.20 |
| F5-3-2 | T0 | 99.44 | ND | 8.497 | 40.1 | 53.2 | 6.9 | 0.40 | 0.41 |
| | Shaking at 25°C, 300 rpm for 3 days | 99.19 | 0.18 | 8.498 | 39.4 | 54.2 | 6.3 | 0.40 | 0.41 |
| | Shaking at 25°C, 300 rpm for 7 days | 98.88 | 0.30 | 8.500 | 37.9 | 55.7 | 6.4 | 0.40 | 0.40 |
| F5-3-3 | T0 | 99.60 | 0.40 | 8.496 | 38.5 | 54.7 | 6.8 | 0.60 | 0.59 |
| | Shaking at 25°C, 300 rpm for 3 days | 99.35 | 0.65 | 8.503 | 38.8 | 54.7 | 6.5 | 0.60 | 0.60 |
| | Shaking at 25°C, 300 rpm for 7 days | 99.35 | 0.65 | 8.503 | 39.7 | 53.8 | 6.4 | 0.60 | 0.60 |
| F5-3-4 | T0 | 99.67 | 0.33 | 8.497 | 37.8 | 55.4 | 6.7 | 0.80 | 0.78 |
| | Shaking at 25°C, 300 rpm for 3 days | 99.54 | 0.46 | 8.501 | 40.2 | 53.4 | 6.3 | 0.80 | 0.78 |
| | Shaking at 25°C, 300 rpm for 7 days | 99.49 | 0.51 | 8.500 | 38.7 | 54.6 | 6.7 | 0.80 | 0.78 |
| F5-3-5 | T0 | 98.81 | 1.19 | 8.497 | 40.0 | 53.3 | 6.6 | 0.20 | 0.25 |
| | Shaking at 25°C, 300 rpm for 3 days | 99.56 | 0.44 | 8.501 | 37.2 | 56.3 | 6.5 | 0.20 | 0.25 |
| | Shaking at 25°C, 300 rpm for 7 days | 99.38 | 0.62 | 8.497 | 40.8 | 52.9 | 6.4 | 0.20 | 0.26 |
| F5-3-6 | T0 | 98.28 | 1.72 | 8.497 | 39.9 | 53.4 | 6.8 | 0.40 | 0.38 |
| | Shaking at 25°C, 300 rpm for 3 days | 99.44 | 0.56 | 8.500 | 37.5 | 56.0 | 6.6 | 0.40 | 0.38 |
| | Shaking at 25°C, 300 rpm for 7 days | 99.13 | 0.87 | 8.496 | 37.7 | 56.0 | 6.5 | 0.40 | 0.40 |
| F5-3-7 | T0 | 96.63 | 3.37 | 8.508 | 38.0 | 55.5 | 6.5 | 0.60 | 0.59 |
| | Shaking at 25°C, 300 rpm for 3 days | 99.35 | 0.65 | 8.501 | 38.8 | 54.5 | 6.6 | 0.60 | 0.60 |
| | Shaking at 25°C, 300 rpm for 7 days | 99.24 | 0.76 | 8.497 | 39.2 | 54.7 | 6.3 | 0.60 | 0.59 |
| F5-3-8 | T0 | 96.07 | 3.93 | 8.496 | 39.6 | 53.8 | 6.7 | 0.80 | 0.84 |
| | Shaking at 25°C, 300 rpm for 3 days | 99.17 | 0.83 | 8.501 | 38.3 | 55.3 | 6.4 | 0.80 | 0.84 |
| | Shaking at 25°C, 300 rpm for 7 days | 99.17 | 0.83 | 8.500 | 36.8 | 56.6 | 6.5 | 0.80 | 0.88 |

| | | | | | | | | | |
|---|---|---|---|---|---|---|---|---|---|
| Remark: T0 = starting point of investigation | | | | | | | | | |

### 4.3 Conclusion of surfactant screening

The samples had a concentration of 10 mg/mL, and were subjected to 40°C for 4 weeks and a shaking test at 25°C and 300 rpm for 7 days for surfactant screening. Among them, appearance, pH, concentration: The F5-3-8 formulation shaken at 300 rpm for 7 days at 25°C had a colorless and slightly opalescent appearance with visible feathery particles. For other formulations and other inspection conditions, all formulations were colorless and slightly opalescent liquids without visible particles. The pH and concentration were within the target values without significant changes. There were no obvious changes in the insoluble microparticles.
(1) After investigation at 40°C for 4 weeks, the quality indicators of each formulation showed a downward trend.
   **SEC-HPLC:** Formulations containing polysorbate 80 were slightly better than those containing poloxamer 188.
   **CE-SDS (NR), CE-SDS (R), and iCIEF** had the same downward trend.
   **Binding activity:** The binding activities all dropped below 50%.
(2) After shaking at 300 rpm for 7 days at 25°C, except for the appearance of the F5-3-8 formulation, there was no significant decline in the quality indicators of other formulations.

From the above experimental results, it could be seen that both polysorbate 80 and poloxamer 188 could be used as surfactants in formulations. The formulation containing polysorbate 80 was slightly better than the formulation containing poloxamer 188. The polysorbate 80 within a reasonable concentration range could stabilize antibody preparations, and the concentration range of 0.02%-0.08% was its optimal concentration range. Furthermore, considering the operational space for production and the usage principles of adjuvants, 0.04% polysorbate 80 was selected as the surfactant for the next experiment.

### Example 5. Determination of the content of components in each formulation

### 5.1 Screening scheme for buffer system components

Combined with the buffer system screening results, different components of the buffer system including different contents and different pHs were screened within the scope of the histidine buffer system using the diffusion interaction parameter (kD) in colloidal stability as an indicator. If kD was a positive value, protein molecules repelled each other in this buffer system, indicating that aggregation was not easily formed. The greater the positive value, the stronger the repulsive effect. If kD was a negative value, protein molecules were attracted to each other in this buffer system, indicating that aggregation was easy to form. The greater the absolute value of the negative value, the stronger the attractive effect. The specific scheme is as shown in Table 21.

**Table 21 Screening of content and pH of buffer system component**

| Formulation | Formulation composition and pH | Detection item |
|---|---|---|
| F4-A | 10 mM histidine-histidine hydrochloride pH 5.5 | |
| F4-B | 5 mM histidine-histidine hydrochloride pH 5.5 | kD |
| F5-A | 10 mM histidine-histidine hydrochloride pH 6.0 | |
| F5-B | 5 mM histidine-histidine hydrochloride pH 6.0 | |

### 5.2 Results of buffer system component screening

The main results of buffer system component screening are summarized in Table 22.

**Table 22 Results of buffer system component screening**

| Formulation | Formulation composition and pH | kD (mL/g) |
|---|---|---|
| F4-A | 10 mM histidine-histidine hydrochloride pH 5.5 | 5.696 |
| F4-B | 5 mM histidine-histidine hydrochloride pH 5.5 | 26.77 |
| F5-A | 10 mM histidine-histidine hydrochloride pH 6.0 | 4.609 |
| F5-B | 5 mM histidine-histidine hydrochloride pH 6.0 | 10.99 |

### 5.3 Determination of contents of components in buffer system and components in each formulation

### 5.3.1 Conclusion of buffer system component screening

Combining the data of Example 2, it can be confirmed that under the histidine buffer system in the range of 5-20 nM, the stability of the composition met the requirements and it was not easy to form aggregates.

Combined with the buffer system screening results, the buffer system was screened for different component content and different pH within the scope of the histidine buffer system using the diffusion interaction parameter (kD) in colloidal stability as an indicator. The results show that the F4-B formulation had the largest kD. Within this buffer system component content and pH range, protein molecules had the strongest mutual repulsion and were least likely to form aggregates. Therefore, we finally selected the F4-B formulation (5 mM histidine-histidine hydrochloride pH 5.5) as the optimal buffer system formulation.

### 5.3.2 Determination of the content of components in each formulation

Based on the adjustment of the content of the buffer system component, and taking into account the convenience of industrial production and medication safety, the content of the adjuvant (trehalose) was adjusted to 9.5% (w/v), and the concentration of the surfactant remained unchanged. The final formulation was 5 mM histidine-histidine hydrochloride, 9.5% trehalose (w/v), 0.04% polysorbate 80 (w/v) pH 5.5. As shown in Table 23, in the final formulation, the osmotic pressure results were within the quality standard range of 290 ± 50 mOsmol/kg.

### Example 6. Preparation method of Tri-52 lyophilized preparation of final formulation

The Tri-52 solution with 10 mg/mL protein concentration in final formulation was aseptically dispensed into vials which was then half-stoppered and lyophilized to obtain a Tri-52 lyophilized preparation. In this example, a vacuum freeze dryer was used to freeze-dry the Tri-52 solution. The freeze-drying steps comprise: (1) Cooling the drying oven of the vacuum freeze dryer to -45°C which was kept for 3 to 5 h; (2) Raising the temperature of the drying oven to - 15°C which was kept for 3 to 5 h; (3) Cooling the temperature of the drying oven to -45°C which was kept for 3 to 5 h, during the pre-freezing process, keeping the heating and cooling rate at 0.5°C to 1°C/min; (4) performing primary drying at a vacuum degree of 0.1 mBar and a temperature of -30 to -20°C, the total time of this step should be greater than or equal to 28 h; (5) After primary drying, increasing the temperature to 25°C at a heating rate of 0.1 to 0.3°C/min to start analytical drying, keeping the vacuum degree at 0.1 mBar for 1 to 2 h, then ultimate vacuum, keeping the temperature unchanged, and the holding time was greater than or equal to 15 h. Among them, steps (1) to (3) were pre-freezing, (4) was primary drying and (5) was analytical drying.

### Example 7. Stability test of final formulation

The Tri-52 lyophilized preparation was prepared using the preparation method of Example 6, and the accelerated stability test at 40°C was conducted. Appearance, reconstitution time, osmotic pressure molar concentration, pH, concentration, moisture, purity, charge isomerism, insoluble microparticles, activity and other indicators were mainly included. Based on the screening results of buffer systems, adjuvants, surfactants and buffer system component content and pH, 1 formulation was finally determined: 10 mg/mL Tri-52, 5 mM histidine-histidine hydrochloride, 9.5% trehalose (W/V), 0.04% polysorbate 80 (W/V), pH 5.5, and dispensed into vials and lyophilized. A sealed packaging system was formed using rubber stoppers and aluminum-plastic lids, and a stability test was conducted at 40°C for 4 weeks.

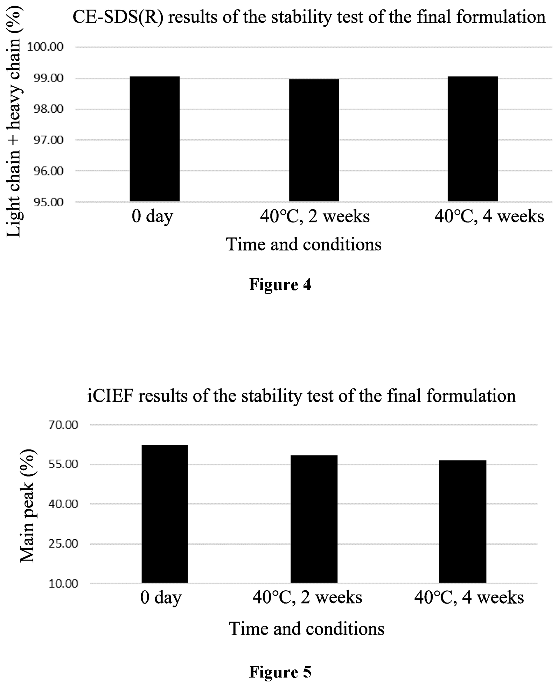

The results are shown in Table 23, Table 24 and Table 25, and Figs. 2 to 5.

**Table 23 Result-1 of stability test of final formulation**

| Investi gation conditi ons | Appearance | | Reco nstit ution time (s) | Protei n conce ntratio n (mg/m L) | Particl e size (nm) | pH | Osmot ic pressu re molar conce ntratio n (mOs mol/kg ) | Insoluble microparticle s (particles/bo ttle) | |
|---|---|---|---|---|---|---|---|---|---|
| | Before recons titutio n | After reconstitution | | | | | | ≥ 10 µm | ≥ 25 µm |
| T0 | White, lumpy, loose body | Colorless, slight opalescence, no visible particles | 30 | 10.33 | 11.13 | 5.531 | 291 | 100 | 14 |
| 40°C, 2 weeks | White, lumpy, loose body | Colorless, slight opalescence, no visible particles | 42 | 10.40 | 10.84 | 5.550 | 291 | 345 | 0 |
| 40°C, 4 weeks | White, lumpy, loose body | Colorless, slight opalescence, no visible particles | 23 | 9.80 | 10.56 | 5.505 | 277 | 19 | 0 |

| | | | | | | | | | |
|---|---|---|---|---|---|---|---|---|---|
| Remark: T0 = starting point of investigation; N/A = not applicable | | | | | | | | | |

**Table 24 Result-2 of stability test of final formulation**

| Investigation conditions | SEC-HPLC(%) | | | CE-SDS (NR) (%) | | | CE-SDS (R) (%) | |
|---|---|---|---|---|---|---|---|---|
| | Low molecular weight peak | Main peak | High molecular weight peak | Low molecular weight peak | Main peak | High molecular weight peak | Main peak | Others |
| T0 | 0.6 | 99.1 | 0.4 | 1.59 | 97.83 | 0.58 | 99.07 | 0.93 |
| 40°C, 2 weeks | 0.7 | 99.0 | 0.3 | 1.30 | 97.90 | 0.79 | 98.97 | 1.03 |
| 40°C, 4 weeks | 0.60 | 98.92 | 0.48 | 1.29 | 97.89 | 0.82 | 99.07 | 0.93 |

| | | | | | | | | |
|---|---|---|---|---|---|---|---|---|
| Remark: T0 = starting point of investigation | | | | | | | | |

**Table 25 Result-3 of stability test of final formulation**

| Investigation conditions | Activity% | Moisture% | iCIEF | | | |
|---|---|---|---|---|---|---|
| | | | Isoelectric point | Acidic peak | Main peak | Basic peak |
| T0 | 100 | 0.83 | 8.3 | 34.2 | 62.5 | 3.4 |
| 40°C, 2 weeks | 97 | 1.66 | 8.5 | 34.0 | 58.4 | 7.6 |
| 40°C, 4 weeks | 101 | 2.01 | 8.50 | 34.2 | 56.6 | 9.2 |

| | | | | | | |
|---|---|---|---|---|---|---|
| Remark: T0 = starting point of investigation | | | | | | |

After accelerated stability test at 40°C for 4 weeks, there were no significant changes in appearance, reconstitution time, osmotic pressure molar concentration, pH, concentration, SEC-HPLC, CE-SDS (NR), CE-SDS (R), insoluble microparticles, and activity, moisture was ≤ 3%, the main peak of iCIEF decreased slightly in terms of charge isomerism, but charge isomerism did not affect the activity. It showed that the protein in this formulation had good stability.

## Claims

1. A composition comprising a multispecific antibody, which is a lyophilization preparation liquid, wherein the multispecific antibody comprises:
(1) a first antigen-binding moiety that binds to tumor cell BCMA antigen;
(2) a second antigen-binding moiety that binds to tumor cell GPRC5D antigen;
(3) a third antigen-binding moiety that binds to immune cell CD3 antigen.

2. The composition of claim 1, wherein the multispecific antibody comprises:
(1) the first antigen-binding moiety that binds to BCMA comprising HCDR1, HCDR2 and HCDR3 of VHH, wherein the HCDR1 comprises a sequence as shown in SEQ ID NO.2, the HCDR2 comprises a sequence as shown in SEQ ID NO.3, and the HCDR3 comprises a sequence as shown in SEQ ID NO.4;
(2) the second antigen-binding moiety that binds to GPRC5D comprising a heavy chain variable region and a light chain variable region of the antibody or a fragment thereof, wherein the heavy chain variable region comprises HCDR1 with a sequence as shown in SEQ ID NO. 6, HCDR2 with a sequence as shown in SEQ ID NO.7, and HCDR3 with a sequence as shown in SEQ ID NO.8; and the light chain variable region comprises LCDR1 with a sequence as shown in SEQ ID NO.10, LCDR2 with a sequence as shown in SEQ ID NO.11, and LCDR3 with a sequence as shown in SEQ ID NO.12; and
(3) the third antigen-binding moiety that binds to CD3 comprising a heavy chain variable region and a light chain variable region of the antibody or a fragment thereof, wherein the heavy chain variable region comprises HCDR1 with a sequence as shown in SEQ ID NO.14, HCDR2 with a sequence as shown in SEQ ID NO.15, and HCDR3 with a sequence as shown in SEQ ID NO.16; and the light chain variable region comprises LCDR1 with a sequence as shown in SEQ ID NO.18, LCDR2 with a sequence as shown in SEQ ID NO.19, and LCDR3 with a sequence as shown in SEQ ID NO.20.

3. The composition of claim 2, wherein the multispecific antibody comprises:
(1) the first antigen-binding moiety that binds to BCMA comprising VHH with a sequence as shown in SEQ ID NO.1;
(2) the second antigen-binding moiety that binds to GPRC5D comprising a heavy chain variable region with a sequence as shown in SEQ ID NO.5 and a light chain variable region with a sequence as shown in SEQ ID NO.9; and
(3) the third antigen-binding moiety that binds to CD3 comprising a heavy chain variable region with a sequence as shown in SEQ ID NO.13 and a light chain variable region with a sequence as shown in SEQ ID NO.17.

4. The composition of claim 2 or 3, wherein the multispecific antibody comprises an Fc domain of human IgG4.

5. The composition of claim 4, wherein the Fc domain of human IgG4 comprises Fc1 and Fc2 which are different.

6. The composition of claim 5, wherein the Fc1 and the Fc2 which are different comprise an amino acid mutation that changes an effector function, wherein the mutation that changes the effector function comprises an S228P, F234A and/or L235A mutation of IgG4 Fc.

7. The composition of claim 6, wherein the Fc1 and the Fc2 which are different comprise a heterodimerization-promoting amino acid mutation; preferably, the heterodimerization-promoting amino acid mutation comprises a knob-into-hole (KIH) mutation; and more preferably, the knob-into-hole mutation comprises: (a) a T366W mutation in either Fc region; and (b) a T366S, L368A and/or Y407V mutation in the other Fc region.

8. The composition of claim 7, wherein the multispecific antibody comprises a first heavy chain with a sequence as shown in SEQ ID NO.21, a second heavy chain with a sequence as shown in SEQ ID NO.22, a first light chain with a sequence as shown in SEQ ID NO.23 and a second light chain with a sequence as shown in SEQ ID NO.23.

9. The composition of claim 8, wherein the either Fc region further comprises a His-tag, and the other Fc region further comprises a Flag-tag.

10. The composition of claim 1, wherein the multispecific antibody comprises a first light chain, a second light chain, a first heavy chain and a second heavy chain, wherein:
(1) the first light chain and the second light chain are the same and have the structure as shown below: VLₐ-CL; and
(2) the first heavy chain and the second heavy chain are different and have the structure as shown below: VHH_{b}-L1-VHₐ-CH1-Fc1 and VHₐ-CH1-L2-VH_{CD3}-L3-VL_{CD3}-Fc2, respectively; optionally, the carboxyl terminus of one heavy chain further has a Flag tag and/or the carboxyl terminus of the other heavy chain further has a His tag;
wherein VHₐ and VLₐ are a heavy chain variable region and a light chain variable region that specifically bind to GPRC5D, respectively, VHH_{b} is a nanobody that specifically binds to BCMA, VH_{CD3} and VL_{CD3} are a heavy chain variable region and a light chain variable region that specifically bind to CD3, respectively, and L1, L2 and L3 are the same or different linkers, respectively.

11. The composition of claim 10, wherein the Fc1 and the Fc2 of the multispecific antibody are different, the Fc1 is knob-Fc, and the Fc2 is hole-Fc; or, the Fc1 is hole-Fc, and the Fc2 is knob-Fc; preferably, wherein the knob-Fc comprises a T366W mutation, and/or the hole-Fc comprises a T366S, L368A and/or Y407V mutation; preferably, the Fc1 and the Fc2 are the Fc regions of IgG4; and more preferably, the Fc1 is knob-Fc, and the Fc2 is hole-Fc.

12. The composition of claim 11, wherein the Fc1 and the Fc2 of the multispecific antibody are different and have an amino acid mutation that changes an effector function; preferably, the mutation that changes the effector function comprises an S228P, F234A and/or L235A mutation of IgG4 Fc.

13. The composition of any one of claims 1 to 12, wherein the composition further comprises a buffer system and a surfactant.

14. The composition of claim 13, wherein the buffer system comprises a citrate buffer solution, a histidine buffer solution and/or a phosphate buffer solution.

15. The composition of claim 14, wherein the buffer system can comprise a histidine-histidine hydrochloride buffer solution, and the histidine- histidine hydrochloride buffer solution has a content of about 5 to 20 mM.

16. The composition of claim 14, wherein the buffer system can comprise a histidine-histidine hydrochloride buffer solution, and the histidine-hydrochloride buffer solution has a content of about 5 mM, about 10 mM, or about 20 mM.

17. The composition of claim 13, wherein the surfactant comprises polysorbate 80 and/or poloxamer 188.

18. The composition of claim 17, wherein the surfactant comprises polysorbate 80, and the polysorbate 80 can have a mass concentration content of about 0.02% to 0.08%.

19. The composition of claim 17, wherein the surfactant comprises polysorbate 80, and the polysorbate 80 can have a mass concentration content of about 0.2%, 0.4%, 0.6% or 0.8%.

20. The composition of any one of claims 1 to 19, wherein the composition further comprises a stabilizer, and the stabilizer comprises glycine, sucrose, trehalose and/or mannitol.

21. The composition of claim 20, wherein the stabilizer comprises trehalose, and the trehalose has a content of about 8.0% to 9.5% (w/v).

22. The composition of claim 21, wherein the stabilizer comprises trehalose, and the trehalose has a content of about 8%, about 8.5%, about 9%, or about 9.5% (w/v).

23. The composition of any one of claims 1 to 22, wherein the composition has a pH of about 5.0 to 7.0; preferably, the composition has a pH of about 5.5 to 6.0; and more preferably, the composition has a pH of about 5.5.

24. The composition of any one of claims 1 to 23, comprising:
(1) a multispecific antibody;
(2) a histidine-histidine hydrochloride buffer system at about 5 mM to 20 mM;
(3) polysorbate 80 at about 0.02% to 0.08% mass concentration; and
(4) trehalose at about 8.0% to 9.5% mass concentration;
wherein the composition has a pH of about 5.0 to 7.5.

25. The composition of claim 24, wherein the composition comprises:
(1) a multispecific antibody;
(2) a histidine-histidine hydrochloride buffer system at about 5 mM;
(3) polysorbate 80 at about 0.04% (w/v); and
(4) trehalose at about 9.5% (w/v);
wherein the composition has a pH of about 5.5.

26. The composition of any one of claims 1 to 25, wherein the composition further comprises a solvent, and the solvent comprises a sterile pure water and/or water for injection.

27. A preparation method for the composition of any one of claims 1 to 26, wherein the preparation method comprises the following steps:
(1) thoroughly mixing the buffer component, the surfactant and the stablizer with the solvent to obtain a mixed solution;
(2) thoroughly mixing the multispecific antibody molecule and the mixed solution;
optionally, the preparation method further comprises steps of sterilization and pyrogen removal.

28. A lyophilized powder preparation, wherein the lyophilized powder is obtained by lyophilizing the composition of any one of claims 1 to 26; optionally, before lyophilizing, steps of sterilization and pyrogen removal of the composition are further comprised.

29. The lyophilized powder preparation of claim 28, wherein the lyophilized powder preparation is used for subcutaneous injection in a patient after being reconstituted.

30. A method for preparing a lyophilized powder preparation, wherein the method comprises lyophilizing the composition prepared according to the preparation method of claim 27.

31. Use of the composition of any one of claims 1 to 26 or the lyophilized powder preparation of claim 28 or 29 in the preparation of a drug for treating a tumor, wherein preferably, the tumor is B-cell lymphoma; more preferably, the B-cell lymphoma is multiple myeloma (MM); and most preferably, the multiple myeloma is relapsed/refractory multiple myeloma (R/RMM).

32. Use of the composition of any one of claims 1 to 26 or the lyophilized powder preparation of claim 28 or 29 in the treatment of a subject suffering from a tumor, wherein preferably, the tumor is B-cell lymphoma; more preferably, the B-cell lymphoma is multiple myeloma (MM); and most preferably, the multiple myeloma is relapsed/refractory multiple myeloma (R/RMM).

33. A method for treating a tumor in a subject, comprising administering the composition according to any one of claims 1 to 26 or the lyophilized powder formulation according to claim 28 or 29 to the subject, wherein preferably, the tumor is a B-cell lymphoma; more preferably, the B-cell lymphoma is multiple myeloma (MM); and most preferably, the multiple myeloma is relapsed/refractory multiple myeloma (R/R MM).
